# EUROPEAN PATENT APPLICATION

(11) **EP 4 678 128 A1**
(43) Date of publication of application: **14.01.2026**
(21) Application number: 25188310.4
(22) Date of filing: 09.07.2025
(51) Int. Cl.: A61B 18/14, A61B 5/287, A61B 18/00, A61B 34/20

(54) **ENCAPSULATED CATHETER WITH FRAMEWORK**

(30) Priority: 10.07.2024 US 202463669344 P; 18.06.2025 US 202519242536
(71) Applicant: Biosense Webster (Israel) Ltd., 2066717 Yokneam (IL)
(72) Inventor: EBRAHIMI, Babak, Irvine, 92618 (US); RODRIGUEZ SOTO, Juan, Irvine, 92618 (US); VAN NIEKERK, Pieter Emmelius, Irvine, 92618 (US); ABBAS, Mohammad, Irvine, 92618 (US)
(74) Representative: Carpmaels & Ransford LLP

(57) **Abstract**

The disclosed technology includes a framework for an end effector of a medical device. The framework includes a base, a first cantilevered spine, a second cantilevered spine, and a first support. The base connects with an elongated shaft of the medical device. The first cantilevered spine extends from the base along a longitudinal axis to a terminal portion. The second cantilevered spine extends from the base along the longitudinal axis to a terminal portion. The first support connects the terminal portion of the first cantilevered spine with an intermediate portion of the second cantilevered spine.

## Description

### CROSS REFERENCE TO RELATED APPLICATIONS

This application claims the benefit of priority under 35 U.S.C. § 119 to prior filed U.S. Provisional Patent Application No. 63/669,344, filed July 10, 2024 (Attorney Docket No.: 253757.000498 (BIO6936USPSP1)), the entire contents of which is hereby incorporated by reference as if set forth in full herein.

### FIELD

The present technology relates generally to medical devices, and in particular medical probes with electrodes, and further relates to, but not exclusively, medical probes suitable for use to map and/or ablate tissue.

### BACKGROUND

Cardiac arrhythmia, such as atrial fibrillation, occurs when regions of cardiac tissue abnormally conduct electric signals to adjacent tissue, thereby disrupting the normal cardiac cycle and causing asynchronous rhythm. Sources of undesired signals can be located in tissue of an atria or a ventricle. Unwanted signals are conducted elsewhere through heart tissue where they can initiate or continue arrhythmia.

Procedures for treating arrhythmia include surgically disrupting the origin of the signals causing the arrhythmia, as well as disrupting the conducting pathway for such signals. More recently, it has been found that by mapping the electrical properties of the endocardium and the heart volume, and selectively ablating cardiac tissue by application of energy, it is possible to cease or modify the propagation of unwanted electrical signals from one portion of the heart to another. The ablation process destroys the unwanted electrical pathways by formation of non-conducting lesions.

In this two-step procedure, which includes mapping followed by ablation, electrical activity at points in the heart is typically sensed and measured by advancing a catheter containing one or more electrical sensors into the heart and acquiring data at multiple points. These data are then utilized to select the target areas at which ablation is to be performed.

For greater mapping resolution, it is desirable for a mapping catheter to conform closely to the target anatomy. For mapping within an atria or a ventricle (for example, an apex of a ventricle), it is desirable for a catheter to collect larger amounts of data signals within shorter time spans. It is also desirable for such a catheter to be capable of allowing sufficient electrode contact with different tissue surfaces, for example, flat, curved, irregular or nonplanar surface tissue, and be collapsible for atraumatic advancement and withdrawal through a patient's vasculature. Existing catheters generally require stiff internal structural members to ensure that a predetermined configuration is maintained. The stiffness is a disadvantage during manipulation in the body organ as it can prevent electrodes from contacting the tissue.

Other catheters can include flexible end effectors designed to overcome this disadvantage. These catheters can include layered components that can be time-consuming, complex, and expensive to manufacture and assemble. Moreover, electrical traces and other components associated therewith can be prone to breakage and/or delamination when in use.

### SUMMARY

There is provided, in accordance with the disclosed technology, a framework for an end effector of a medical device. The framework includes a base, a first cantilevered spine, a second cantilevered spine, and a first support. The base is configured to connect with an elongated shaft of the medical device and extends along a longitudinal axis in a distal direction. The first cantilevered spine extends from the base along the longitudinal axis to a terminal portion. The second cantilevered spine extends from the base along the longitudinal axis to a terminal portion. The first support connects the terminal portion of the first cantilevered spine with an intermediate portion of the second cantilevered spine.

The is further provided, in accordance with the disclosed technology, an end effector for a catheter. The end effector includes a framework, a flexible circuit, and an insulative material. The framework includes a base and a plurality of spines extending from the base along a longitudinal axis. The flexible circuit includes a plurality of segments extending along the longitudinal axis, the plurality of segments defining an area not covered by any material, and a plurality of electrodes disposed on each of the segments. The insulative material is disposed between the framework and the flexible circuit so that the framework is spaced apart from the flexible circuit.

There is further provided, in accordance with the disclosed technology, an end effector for a catheter. The end effector includes a framework including a base and a plurality of spines extending from the base along a longitudinal axis. The end effector includes a position sensor spaced apart from the framework, the position sensor including a plurality of location sensing loops. The location sensing loops include a pair of side loops disposed generally symmetrical about the longitudinal axis. Each side loop extends in a loop from a proximal portion of the framework, to a distal portion of the framework, and back to the proximal portion of the framework along the longitudinal axis.

There is further provided, in accordance with the disclosed technology, a flexible circuit. The flexible circuit includes a substrate layer including a stretchable polymer material. The flexible circuit includes one or more conductive traces connected to the substrate layer and configured to conduct current. The flexible circuit includes one or more strain reduction features connected to the substrate layer. Each strain reduction feature includes one of: a sacrificial trace connected to the substrate layer, a heat sink connected to a base of the substrate layer, or a localized stiffener connected to the substrate layer in a region in which a portion of the one or more conductive traces extend.

There is further provided, in accordance with the disclosed technology, an end effector for a catheter. The end effector includes a framework, a flexible circuit, and an insulative material. The framework includes a plurality of spines extending along a longitudinal axis. The flexible circuit includes a substrate layer including a stretchable polymer material, a plurality of conductive traces connected to the substrate layer and configured to conduct current, a plurality of electrodes connected to the substrate layer, a respective conductive trace of the plurality of conductive traces being connected to a respective electrode of the plurality of electrodes, and one or more strain reduction features connected to the substrate layer. Each strain reduction feature includes one of (i) a sacrificial trace connected to the substrate layer, (ii) a heat sink connected to a base of the substrate layer, or (iii) a localized stiffener connected to the substrate layer in a region in which a portion of one or more of the plurality of conductive traces extend. The insulative material at least partially envelops the framework and the flexible circuit.

There is further provided, in accordance with the disclosed technology, a method of manufacturing an end effector for a medical device. The method includes forming a framework that is substantially planar along a longitudinal axis. The method includes forming a flexible circuit, including the steps of forming a substrate layer comprising a stretchable polymer material and providing one or more conductive traces on the substrate layer. The method includes providing one or more strain reduction features on the substrate layer of the flexible circuit. The method includes disposing the flexible circuit over the framework. The method includes heating an insulative material. The method includes reflowing the insulative material such that the insulative material envelopes the framework and the flexible circuit.

There is further provided, in accordance with the disclosed technology, a flexible circuit extending along a longitudinal axis. The flexible circuit includes a substrate layer and a plurality of conductive traces. The conductive traces are connected to the substrate layer and are configured to conduct current. Each conductive trace includes a serpentine shape comprising a plurality of arc sections, with each arc section defining a pair of voids with oppositely facing open ends. Each conductive trace generally extends along the longitudinal axis adjacent to one or more conductive traces of the plurality of conductive traces such that each arc section of any respective conductive trace of the plurality of traces is laterally spaced from the voids of the arc sections of the adjacent one or more conductive traces.

### BRIEF DESCRIPTION OF THE DRAWINGS

FIG. 1 is a schematic pictorial illustration of a medical system including a medical device that includes a medical probe with an end effector with electrodes, in accordance with the disclosed technology;
FIG. 2 is a schematic pictorial illustration showing an exploded perspective view of a first exemplary end effector, in accordance with the disclosed technology;
FIG. 3A is a schematic pictorial illustration showing a plan view of a framework of the first end effector, in accordance with the disclosed technology;
FIG. 3B is a schematic pictorial illustration showing a plan view of a position sensor of the first end effector, in accordance with the disclosed technology;
FIG. 3C is a schematic pictorial illustration showing a plan view of an alternative position sensor, similar to the position sensor of FIG. 3B, disposed on the framework of FIG. 3A, in accordance with the disclosed technology;
FIG. 3D is a schematic pictorial illustration showing a plan view of the position sensor of FIG. 3B disposed on the framework of FIG. 3A, in accordance with the disclosed technology;
FIG. 3E is a schematic pictorial illustration showing a plan view of the first end effector, in accordance with the disclosed technology;
FIG. 4 is a schematic pictorial illustration showing a plan view, opposite the view of FIG. 3E, showing the framework of FIG. 3A and the position sensor of FIG. 3B disposed on the flexible circuit of FIG. 3E, with the position sensor of FIG. 3B shown in phantom lines, in accordance with the disclosed technology;
FIG. 5A is a schematic pictorial illustration showing a plan view of a framework and a position sensor disposed on a flexible circuit of a second exemplary end effector, in accordance with the disclosed technology;
FIG. 5B is a schematic pictorial illustration showing a plan view of the position sensor of the second end effector, in accordance with the disclosed technology;
FIG. 5C is a schematic pictorial illustration showing a plan view of a framework and a position sensor disposed on a flexible circuit of a modified second exemplary end effector, in accordance with the disclosed technology;
FIG. 6A is a schematic pictorial illustration showing a plan view of a third exemplary end effector, in accordance with the disclosed technology;
FIG. 6B is a schematic pictorial illustration showing a plan view of a framework and a position sensor disposed on a flexible circuit of the third exemplary end effector, in accordance with the disclosed technology;
FIG. 6C is a schematic pictorial illustration showing a plan view of the position sensor of the third end effector, in accordance with the disclosed technology;
FIG. 7A is a schematic pictorial illustration showing a plan view of a fourth exemplary end effector, in accordance with the disclosed technology;
FIG. 7B is a schematic pictorial illustration showing a position sensor of the fourth end effector in solid lines and other sub-components in phantom lines, in accordance with the disclosed technology;
FIG. 7C is a schematic pictorial illustration showing the position sensor of the fourth end effector, in accordance with the disclosed technology;
FIG. 8A is a schematic pictorial illustration showing a plan view of a flexible circuit, in accordance with the disclosed technology;
FIG. 8B is a schematic pictorial illustration showing a cross-sectional view of the flexible circuit of FIG. 8A, in accordance with the disclosed technology;
FIG. 9A is a schematic pictorial illustration showing a first strain reduction feature for a flexible circuit, in accordance with the disclosed technology;
FIG. 9B is a schematic pictorial illustration showing a cross-sectional view of the flexible circuit of FIG. 9A incorporating the first strain reduction feature, in accordance with the disclosed technology;
FIG. 10A is a schematic pictorial illustration showing a second strain reduction feature for a flexible circuit, in accordance with the disclosed technology;
FIG. 10B is a schematic pictorial illustration showing a cross-sectional view of the flexible circuit of FIG. 10A incorporating the second strain reduction feature, in accordance with the disclosed technology;
FIG. 11 is a schematic pictorial illustration showing a third strain reduction feature for a flexible circuit, in accordance with the disclosed technology;
FIG. 12 is a schematic pictorial illustration showing a cross-sectional view of an end effector incorporating a strain reduction feature, in accordance with the disclosed technology;
FIG. 13 is a flow chart of a method of manufacturing an end effector for a medical device, in accordance with the disclosed technology;
FIG. 14A is a schematic pictorial illustration showing a plan view of another flexible circuit, in accordance with the disclosed technology;
FIG. 14B is a schematic pictorial illustration showing a detail view of Detail A in FIG. 14A, in accordance with the disclosed technology;
FIG. 14C is a schematic pictorial illustration showing a detail view of Detail B in FIG. 14A, in accordance with the disclosed technology; and
FIG. 14D is a schematic pictorial illustration showing a detail view of Detail C in FIG. 14A, in accordance with the disclosed technology.

### DETAILED DESCRIPTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" or "generally" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%. In addition, as used herein, the terms "patient," "host," "user," and "subject" refer to any human or animal subject and are not intended to limit the systems or methods to human use, although use of the subject technology in a human patient represents a preferred embodiment. As well, the term "proximal" indicates a location closer to the operator or physician whereas "distal" indicates a location further away to the operator or physician.

As discussed herein, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "operator" can include a doctor, surgeon, technician, scientist, or any other individual or delivery instrumentation associated with delivery of a multi-electrode catheter for the treatment of drug refractory atrial fibrillation to a subject.

The present disclosure is related to systems, methods, uses, and devices for mapping and ablation of cardiac tissue to treat cardiac arrhythmias. Ablative energies are typically provided to cardiac tissue by a tip portion of a catheter which can deliver ablative energy alongside the tissue to be ablated. Some example catheters include three-dimensional structures at the tip portion and are configured to administer ablative energy from various electrodes positioned on the three-dimensional structures. Ablative procedures incorporating such example catheters can be visualized using fluoroscopy.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation. RF approaches can have risks that can lead to tissue charring, burning, steam pop, phrenic nerve palsy, pulmonary vein stenosis, and esophageal fistula. Cryoablation is an alternative approach to RF ablation that can reduce some thermal risks associated with RF ablation. However maneuvering cryoablation devices and selectively applying cryoablation is generally more challenging compared to RF ablation; therefore, cryoablation is not viable in certain anatomical geometries which may be reached by electrical ablation devices.

The present disclosure can include electrodes configured for RF ablation, cryoablation, and/or irreversible electroporation (IRE). IRE can be referred to throughout this disclosure interchangeably as pulsed electric field (PEF) ablation and pulsed field ablation (PFA). IRE as discussed in this disclosure is a non-thermal cell death technology that can be used for ablation of atrial arrhythmias. To ablate using IRE/PEF, biphasic voltage pulses are applied to disrupt cellular structures of myocardium. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE therefore has the capability to spare adjacent heat sensitive structures or tissues which would be of benefit in the reduction of possible complications known with ablation or isolation modalities. Additionally, or alternatively, monophasic pulses can be utilized.

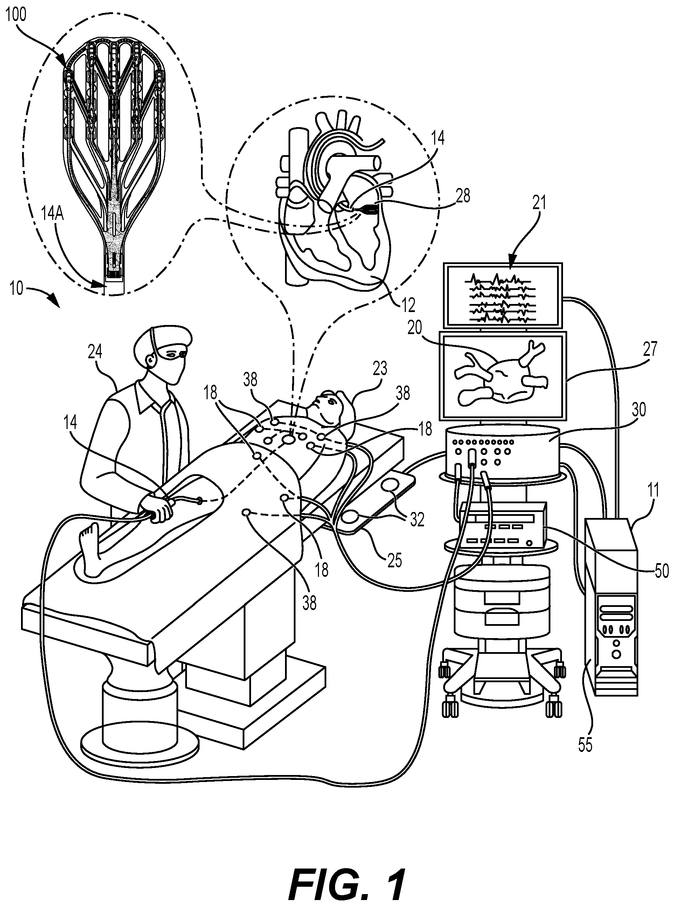

Reference is made to FIG. 1 showing an example catheter-based electrophysiology mapping and ablation system 10. System 10 includes multiple catheters, which are percutaneously inserted by physician 24 through the patient's 23 vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example catheter/medical probe 14 that is configured for sensing IEGM is illustrated herein. Physician 24 brings a catheter shaft with end effector of catheter 14 (i.e., multilayered distal tip/distal tip 28) into contact with the heart wall for sensing a target site in heart 12. For ablation, physician 24 would similarly bring a distal end of an ablation catheter to a target site for ablating.

Catheter 14 is an exemplary catheter that includes one and preferably multiple electrodes 102 optionally distributed over end distal tip 28 coupled to a catheter shaft and configured to sense the IEGM signals as described in more detail below. Catheter 14 may additionally include a position sensor embedded in or near end distal tip 28 for tracking position and orientation of distal tip 28. Optionally and preferably, position sensor is a magnetic based position sensor including multiple magnetic coils for sensing three-dimensional (3D) position and orientation.

Magnetic based position sensor may be operated together with a location pad 25 including a plurality of magnetic coils 32 configured to generate magnetic fields in a predefined working volume. Real time position of end effector 100 of catheter 14 may be tracked based on magnetic fields generated with location pad 25 and sensed by magnetic based position sensor. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091, each of which are incorporated herein by reference.

System 10 includes one or more electrode patches 38 positioned for skin contact on patient 23 to establish location reference for location pad 25 as well as impedance-based tracking of electrodes 102. For impedance-based tracking, electrical current is directed toward electrodes 102 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182, each of which are incorporated herein by reference.

A recorder 11 displays electrograms 21 captured with body surface ECG electrodes 18 and intracardiac electrograms (IEGM) captured with electrodes 102 of catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes 160A, 160B at a end effector of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (4) displaying on display device 27 sites of interest such as places where ablation energy has been applied. One commercial product embodying elements of the system 10 is available as the CARTO^{™} 3 System, available from Biosense Webster, Inc., 31 Technology Drive, Suite 200, Irvine, CA 92618.

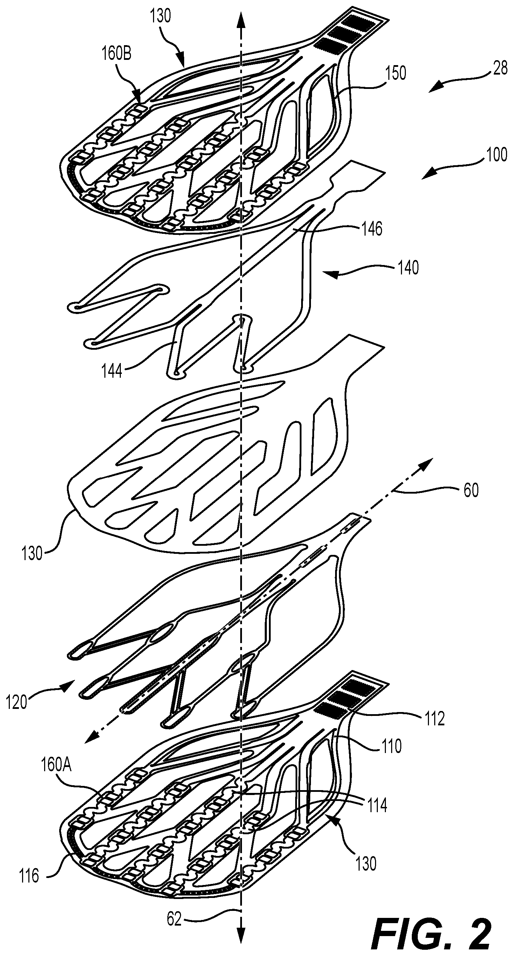

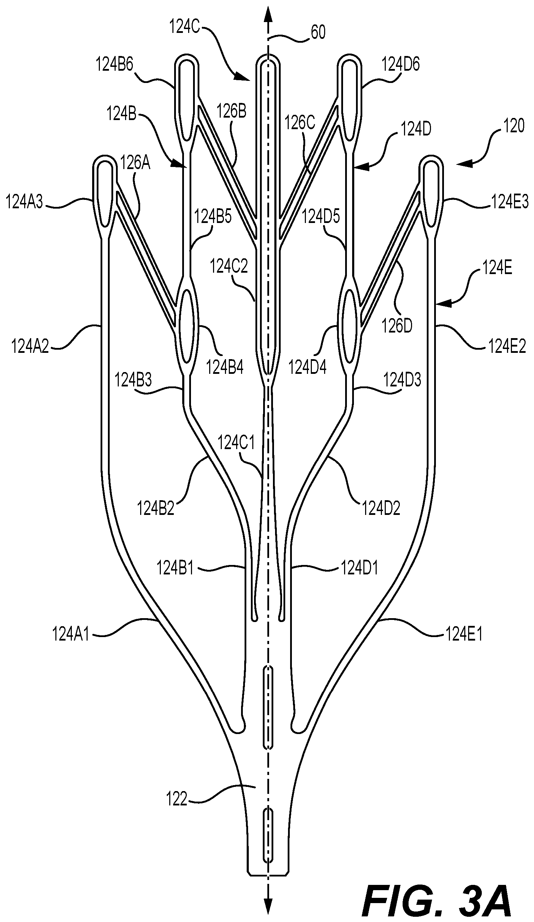

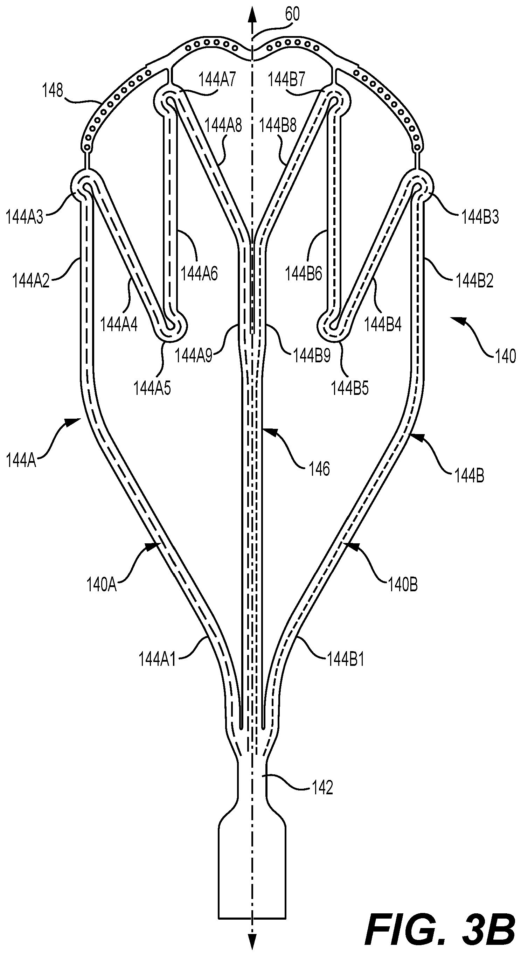

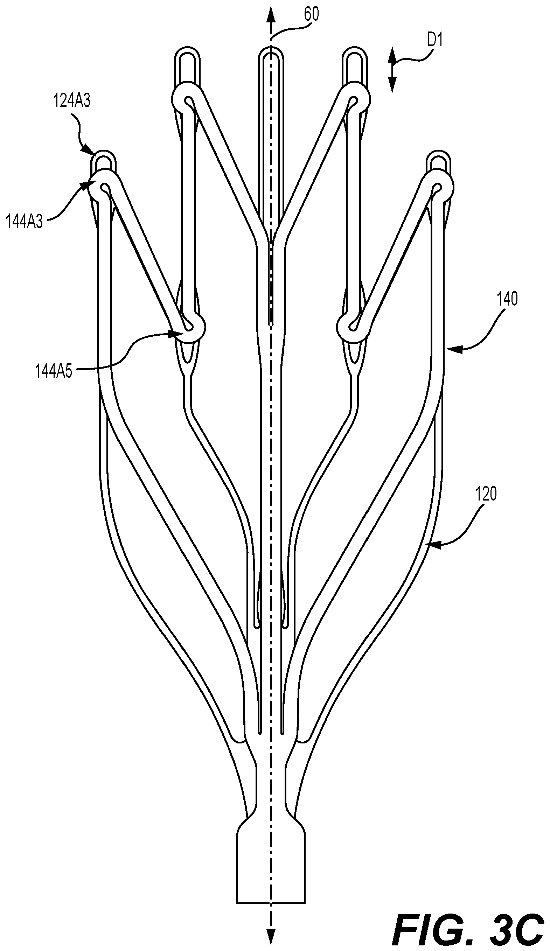

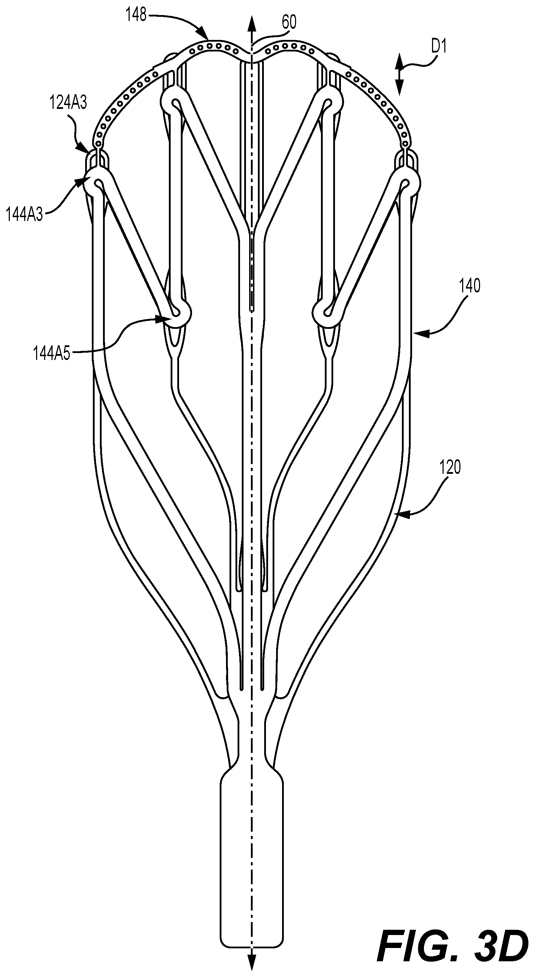

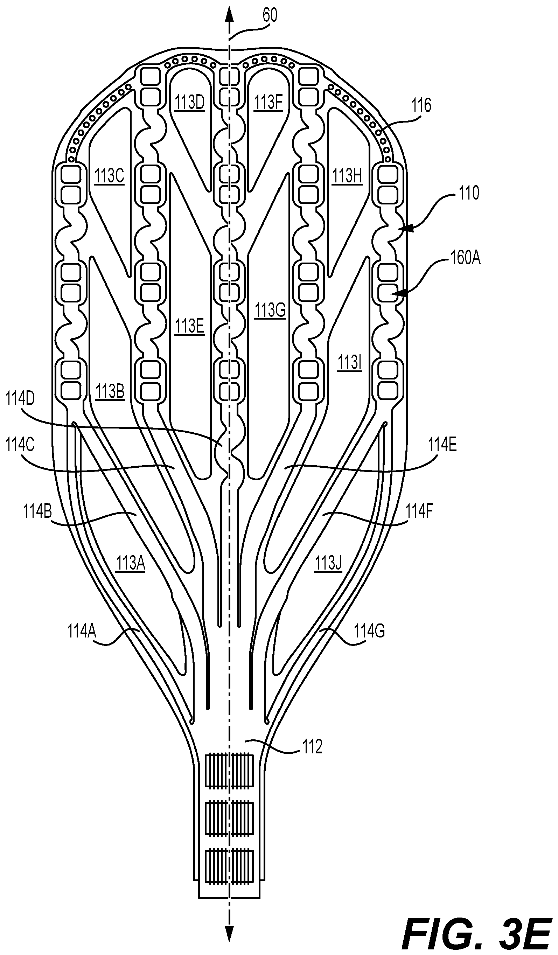

FIGs. 2-3E provide views of one or more portions of a first end effector 100 (the term "end effector" is used synonymously with the term "distal tip" herein) that is configured for insertion into an internal body cavity of a patient. Specifically, FIG. 2 shows an exploded view of the first end effector 100, with the components thereof exploded vertically along vertical axis 62, FIG. 3A shows a framework 120 of the first end effector 100, FIG. 3B shows a position sensor 140 of the first end effector 100, FIG. 3C shows a relative positioning of a distal end of the position sensor 140 and the distal end of the framework 120 (with a bridge 148 removed/omitted), FIG. 3D shows a relative position of the distal end of the position sensor 140 of FIG. 3B (that includes a bridge 148), and FIG. 3E shows a second flexible circuit 150 disposed over the position sensor 140 and the framework 120 (with insulative material 130 obscuring the view of the position sensor 140 and framework 120). An opposing first flexible circuit 110 is identically designed in this example and, therefore, any description of the first flexible circuit 110 also accurately describes the configuration of the second flexible circuit 150, unless specifically noted to the contrary.

The first end effector 100 extends from a proximal end (upper right-hand side of FIG. 2), that connects to an elongated shaft 14A (FIG. 1), to a distal end (bottom left-hand side of FIG. 2) along a longitudinal axis 60. The first flexible circuit 110 (and the second flexible circuit) includes a plurality of electrodes 160A, 160B. In some examples, the term "flexible circuit" includes thin-film circuit, flexible printed circuit board, thin film deposition via lithography and etching processes on substrates such as polyimide, copper, liquid crystal polymer (LCP), nitinol substrate, thermoplastic polyurethane (TPU), silicone, thermoset resin, or other polymeric substrates. In some examples, the flexible circuits described herein can be made primarily of polyimide. In other examples, it can be made of any of biocompatible polyimides, glass-reinforced epoxy laminate materials, copper, or graphene, alone or in combination. In some examples, the electrodes 160A, 160B described herein can include at least one mapping electrode and/or at least one ablation electrode and can be configured to detect electrophysiological signals or transmit ablative energy AC or DC from an energy generator to the tissue according to the various ablation methods previously described e.g., RF, IRE, etc. The structure of the first flexible circuit 110 (and, likewise, the second flexible circuit 150) is discussed in further detail with respect to FIG. 3D below.

The first end effector 100 can further include a framework 120 contiguous to the insulative material 130 or in the insulative material 130. In examples in which the distal tip 28 includes framework 120, the framework 120 can be disposed directly on the first flexible circuit 110 (or both the first flexible circuit 110 and the second flexible circuit 150) with none, or very little, of the insulative material 130 coming between the two. In other examples, insulative layers of the insulative material 130 can space the framework 120 from the flexible circuits 100, 150. In some examples, the framework 120 is disposed in the insulative material 130 and is substantially planar along the longitudinal axis 60 such that the longitudinal axis 60 is parallel to or coincident with the framework 120. In some examples, the framework is symmetric relative to the longitudinal axis 60. In some examples, the framework 120 is formed from a flexible, resilient material. By way of example, the framework can be formed from a shape-memory alloy such as nickel-titanium, also known as Nitinol, cobalt chromium, stainless steel, and/or other alloys that exhibit pseudo-elastic and/or super-elastic properties. The framework 120 can be formed from a planar or cylindrical stock of material using any suitable method. For example, the framework 120 can be formed by cutting, laser cutting, stamping, etc.

Making specific reference to FIG. 3A, the framework 120 includes a base 122, a plurality of spines 124A-E extending therefrom along the longitudinal axis 60, and a plurality of supports 126A-D connecting adjacent spines 124A-E. In some examples, there are five cantilevered spines (i.e., a first cantilevered spine 124A, a second cantilevered spine 124B, a third, central cantilevered spine 124C, a fourth cantilevered spine 124D, and a fifth cantilevered spine 124E); however, other numbers of cantilevered spines can be used without departing from the spirit and scope of the present disclosure. Moreover, it is noted that the spines 124A-E are cantilevered because their distalmost tips/ends are unsupported/unfixed. In some examples, the spines 124A-E can be supported at their distalmost tips/ends (i.e., they are not cantilevered).

The base 122 connects with the elongated shaft 14A and extends along the longitudinal axis 60 in a distal direction from the elongated shaft 14A. The framework base 122 generally aligns with and supports the bases 112 of the flexible circuits 110, 150.

The first cantilevered spine 124A (also referred to herein as the "first spine") extends from the base 112 along the longitudinal axis 60 to an unsupported distalmost end. The first cantilevered spine 124A includes (1) a first segment 124A1 extending at an oblique angle, relative to the longitudinal axis 60, from the base 112, (2) a second segment 124A2 extending generally parallel to the longitudinal axis 60 from the first segment 124A1, and (3) a third segment forming a terminal portion 124A3 including (i) an eyelet 124A3 and (ii) the unsupported distalmost end of the first cantilevered spine 124A. The eyelet 124A3 aids in supporting other sub-components of the first end effector 100 and is discussed in greater detail below.

The second cantilevered spine 124B (also referred to herein as the "second spine") extends from the base 112 along the longitudinal axis 60 to an unsupported distalmost end. The second cantilevered spine 124B includes (1) a first segment 124B1 extending generally parallel to the longitudinal axis 60 from the base 112, (2) a second segment 124B2 extending at an oblique angle, relative to the longitudinal axis 60, from the first segment 124B1, (3) a third segment 124B3 extending generally parallel to the longitudinal axis 60 from the second segment 124B4, (4) a fourth segment 124B4 including an eyelet 124B4 and extending from the third segment 124B3, (5) a fifth segment 124B5 extending generally parallel to the longitudinal axis 60 from the fourth segment 124B4, and (6) a sixth segment forming a terminal portion 124B6 including (i) another eyelet 124B6 and (ii) the unsupported distalmost end of the first cantilevered spine 124A. The eyelet 124B6 aids in supporting other sub-components of the first end effector 100 (discussed in greater detail below).

The third cantilevered spine 124C (also referred to herein as the "third spine") is centrally located relative two the first/second cantilevered spines 124A, 124B and the fourth/fifth cantilevered spines 124D, 124E. It extends from the base 112 to an unsupported distalmost end. The third cantilevered spine 124C includes (1) a first segment 124C1 extending generally parallel to or co-axial with the longitudinal axis 60 from the base 112 and (2) a second segment forming a terminal portion 124C2 including (i) an eyelet 124C2 that has an elongated shape relative to the aforementioned eyelets 124A3, 124B4, 124B6 and (ii) the unsupported distalmost end. The elongated eyelet 124C2 overlaps with the first eyelet 124B4 and the second eyelet 124B6 of the second cantilevered spine 124B in the distal direction/along the longitudinal axis 60.

The fourth cantilevered spine 124D (also referred to herein as the "fourth spine") extends from the base 112 along the longitudinal axis 60 to an unsupported distalmost end and is generally symmetrical with the second cantilevered spine 124B relative to the longitudinal axis 60. The fourth cantilevered spine 124D includes (1) a first segment 124D1 extending generally parallel to the longitudinal axis 60 from the base 112, (2) a second segment 124D2 extending at an oblique angle, relative to the longitudinal axis 60, from the first segment 124D1, (3) a third segment 124D3 extending generally parallel to the longitudinal axis 60 from the second segment 124D4, (4) a fourth segment 124D4 including an eyelet 124D4 and extending from the third segment 124D3, (5) a fifth segment 124D5 extending generally parallel to the longitudinal axis 60 from the fourth segment 124D4, and (6) a sixth segment forming a terminal portion 124B6 including (i) another eyelet 124D6 and (ii) the unsupported distalmost end of the fourth cantilevered spine 124D. The eyelet 124D3 aids in supporting other sub-components of the first end effector 100 (discussed in greater detail below).

The fifth cantilevered spine 124E (also referred to herein as the "fifth spine") extends from the base 112 along the longitudinal axis 60 to an unsupported distalmost end and is generally symmetrical with the first cantilevered spine 124A relative to the longitudinal axis 60. The fifth cantilevered spine 124E includes (1) a first segment 124E1 extending at an oblique angle, relative to the longitudinal axis 60, from the base 112, (2) a second segment 124E2 extending generally parallel to the longitudinal axis 60 from the first segment 124E1, and (3) a third segment forming a terminal portion 124E3 including (i) an eyelet 124E3 and (ii) the unsupported distalmost end of the fifth cantilevered spine 124E. The eyelet 124E3 aids in supporting other sub-components of the first end effector 100 and is discussed in greater detail below.

As mentioned above, a plurality of supports 126A-D interconnect respective adjacent spines 124A-E. More specifically, the supports include a first support 126A, a second support 126B, a third support 126C, and a fourth support 126D. These supports 126A-D extend at an oblique (i.e., non-perpendicular) angle relative to the longitudinal axis 60 in the distal direction from one spine to another.

The first support 126A connects the eyelet 124A3 (i.e., the terminal portion) of the first spine 124A with an eyelet 124B4 (i.e., an intermediate portion) of the second spine 124B. The second support 126A connects another eyelet 124B6 (i.e., the terminal portion) of the second spine 124B with the elongated eyelet 124C2 (at an intermediate portion thereof) of the third spine 124C. The third support 126C is mirrored symmetrically with the second support 126B and connects the elongated eyelet 124C2 of the third spine 124C with an eyelet 124D6 of the fourth spine 124D. The fourth support 126D is mirrored symmetrically with the first support 126A and connects another eyelet 124D6 of the fourth spine 124D with the eyelet 124E3 of the fifth spine 124E.

As seen particularly in FIG. 3A, the first and second supports 126A, 126B are substantially parallel to one another, which is optimum is the present examples to support the multiple parallel branch segments 114 of the flexible circuits 110, 150 and to reduce stiffness. Additionally, the first segment 124A1 of the first spine 124A and the second segment 124B2 of the second spine 124B are substantially parallel with the first and second supports 126A, 126B. Likewise, the third and fourth supports 126C, 126D are substantially parallel to one another, with the first segment 124E1 of the fifth spine 124E and the second segment 124D2 of the fourth spine 124D also being parallel thereto.

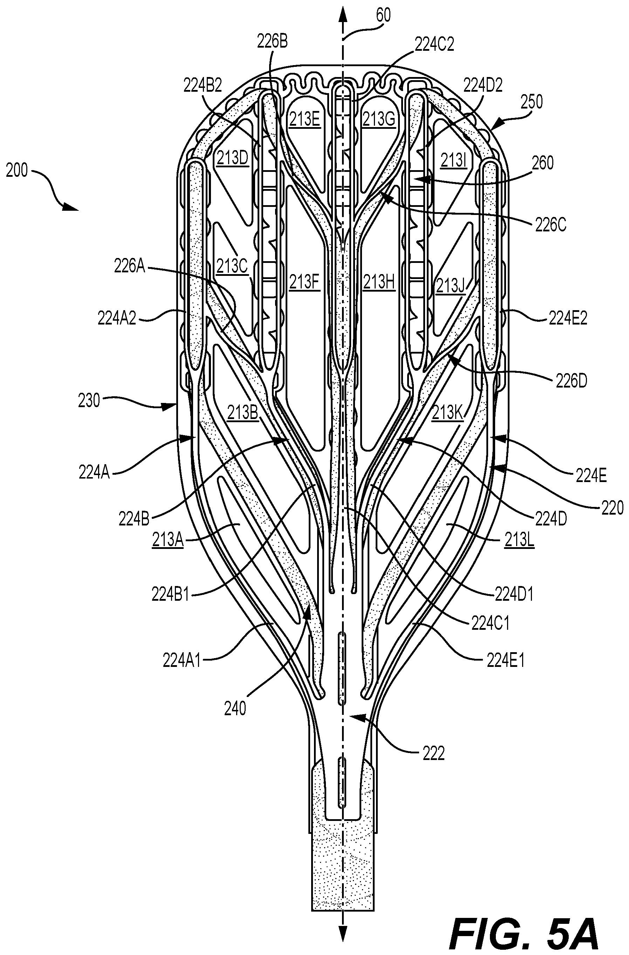

In some examples, each support 126A-E includes a pair of legs that define a void therebetween (similar to an eyelet) and connect respective segments of the spines. In other examples (e.g., the example depicted in FIG. 5A and discussed in greater detail below), the supports 126A-E are singular legs.

Making specific reference to FIGs. 2 and 3B, a position sensor 140 can be provided that is sandwiched between the second flexible circuit 150 (or the first flexible circuit 110) and the framework 120, generally parallel thereto, and spaced therefrom along the vertical axis 62. In this example, the position sensor 140 is configured as a flexible circuit (e.g., similar to flexible circuits 110, 150, but without electrodes formed thereon) that has one or more loops 140A, 140B with traces that each, when subjected to a magnetic field, induce a current indicative of a position of the first end effector 100.

The position sensor 140 includes a base 142, a first side section 144A laterally offset from the longitudinal axis 60, a second side section 144B laterally offset from the longitudinal axis 60, a center segment 146 running along the longitudinal axis 60, and a bridge 148. The bridge 148 primarily functions to add structural integrity to the position sensor 140 during lamination (to prevent movement of the side sections 144A, 144B and center segment 146). Additionally, holes formed in the bridge 148 can act as rivets and/or anchor points for better adhesion to the insulative material 130.

The first side section 144A includes a first segment 144A1 extending from the base 142 obliquely relative the longitudinal axis 60, a second segment 144A2 extending from the first segment 144A1 generally parallel to the longitudinal axis 60, a first first-side curved connecting portion 144A3 connected to the second segment 144A2, a third segment 144A4 connected to and extending from the first first-side curved connecting portion 144A3, a second first-side curved connecting portion 144A5 connected to the third segment 144A4, a fourth segment 144A6 connected to and extending from the second first-side curved connecting portion 144A5, a third first-side curved connecting portion 144A7 connected to the fourth segment 144A6, a fifth segment 144A8 connected to and extending from the third first-side curved connecting portion 144A7, and a sixth segment 144A9 extending from the fifth segment 144A8 and connecting with the center segment 146. In some examples, the second segment 144A2, the fourth segment 144A6, and the sixth segment 144A9 are substantially parallel to one another, while the third segment 144A4 and the fifth segment 144A8 are substantially parallel to one another. In some examples, the segments of the first side section 144B are linear.

The second side section 144B includes a first segment 144B1 extending from the base 142 obliquely relative the longitudinal axis 60, a second segment 144B2 extending from the first segment 144B1 generally parallel to the longitudinal axis 60, a first second-side curved connecting portion 144B3 connected to the second segment 144B2, a third segment 144B4 connected to and extending from the first second side curved connecting portion 144B3, a second second-side curved connecting portion 144B5 connected to the third segment 144B4, a fourth segment 144B6 connected to and extending from the second second-side curved connecting portion 144B5, a third second side curved connecting portion 144B7 connected to the fourth segment 144B6, a fifth segment 144B8 connected to and extending from the third second-side curved connecting portion 144B7, and a sixth segment 144B9 extending from the fifth segment 144B8 and connecting with the center segment 146. In some examples, the second segment 144B2, the fourth segment 144B6, and the sixth segment 144B9 are substantially parallel to one another, while the third segment 144B4 and the fifth segment 144B8 are substantially parallel to one another. In some examples, the segments of the second side section 144B are linear.

The curved connection portions of the position sensor 140 described above have a geometry that aids in forcing the position sensor 140 to fold into a cylindrical shape when inserted into a sheath and/or insertion tool without fracturing. As seen in FIG. 3B, all of the curved connecting portions have an arc shape with an angle equal to or greater than one-hundred and eighty degrees.

Moreover, as particularly shown in FIG. 3C, each curved connecting portion 144A3, 144A5, 144A7, 144B7, 144B5, 144B3 is aligned with a respective eyelet 124A3, 124B4, 124B6, 124D4, 124D6, 124E3 of the framework 120 along the vertical axis 62 of the first end effector 100 such that the eyelets provide additional support to the curved connecting portions. Similarly, the supports 126A-D extend parallel to and aligned with certain segments of the first and second side sections 144A, 144B of the position sensor 140 (e.g., segments 144A4, 144A8, 144B4, and 144B8). It is noted that only a few elements are labeled in FIG. 3C for reference, since all the relevant elements are previously labelled in FIGs. 3A and 3B, respectively, and the intent of FIG. 3C is to illustrate a relative positioning between the position sensor 140 and the framework 120.

As also shown in FIG. 3C, distalmost ends of the first and second side sections 144A, 144B (e.g., curved connecting portions 144A7, 144B7) are spaced from a distalmost end of the framework 120 by a predetermined distance D1. In some examples, the predetermined distance D1 is approximately 1 millimeter, which aids in the position sensor 140 avoiding the distal end of the first end effector 100, which is susceptible to high strain. In some examples, the bridge 148 (FIGs. 3B and 3D) forms a distalmost portion of the position sensor and connects to some of the plurality of curved connecting portions (e.g., portions 144A3, 144A7, 144B3, 144B7). As seen in FIG. 3D, in examples where the bridge 148 is not omitted (e.g., the configuration shown in FIG. 3C), the distalmost ends of the first and second side sections 144A, 144B are still spaced from the distalmost end of the framework 120 by a predetermined distance D1, with the bridge 148 running along or near the framework's distalmost end.

As seen in FIG. 3B and denoted by the evenly sized phantom lines, the position sensor 140 further includes a pair of side location sensing loops 140A (long dash phantom lines), 140B (short dash phantom lines) disposed generally symmetrical about the longitudinal axis 60 that run along the aforementioned side segments 144A1-144A8, 144B1-144B8 and center segment 146 of the position sensor 140. In general, each side loop 140A, 140B extends in a loop from a proximal portion of the framework 120, to a distal portion of the framework 120, and back to the proximal portion of the framework 120 along the longitudinal axis 60 (see FIG. 3C for reference). The first loop 140A extends along the first side section 144A and the center segment 146, while the second loop 140B extends along the second side section 144B and the center segment 146.

As seen in, e.g., FIG. 3E, the flexible circuits 110, 150 are disposed in an insulative material 130 that extends along the longitudinal axis 60 of the plane. The insulative material 130 can be contiguous to the contact surfaces of the electrodes 160 so that only the contact surfaces of at least a portion of the plurality of electrodes 160 are exposed to the ambient environment. As used herein, "ambient environment" refers to the external environment such as the organ in which the first end effector 100 is deployed or in the operating theater prior to being deployed in the biological organ. The insulative material 130 at least partially encapsulates and spaces the different layers of the first end effector 100 (e.g., the flexible circuits 100, 150, the position sensor 140, and the framework 120, which is discussed in greater detail below) along the vertical axis 62.

Insulative material 130 can include one or more sheets fused together proximate the framework 120 into a single, contiguous, generally planar insulative mass 130. This insulative material 130 also serves to enhance the atraumaticity of the end effector tip 100 and to protect the subject from sharp edges. The insulative material 130 can include polymer. The insulative material 130 can be heat formed around at least a portion of the first flexible circuit 110, the second flexible circuit 150, and the framework 120. The polymer can include TPU or other heat formed or shaped material which lends itself to said heat forming.

Furthermore, while the insulative material 130 is shown to be flat in these figures, insulative material 130 can be shaped, scalloped, ribbed, ridged, concaved, convexed, or otherwise configured such that the overall profile of insulative material 130 yields physical and/or mechanical properties, such as rigidity and flexion along multiple axes, required by the distal tip 28, mentioned above.

A flexible substrate of each flexible circuit 110, 150 comprises a bio-compatible material and extends along the plane and has a first side and a second side. In some examples, the flexible substrate is formed entirely from or about entirely from the bio-compatible material. The electrodes 160 are disposed on a surface of the flexible substrate. In some example, the electrodes 160 are disposed on only one side of the substrate. Put another way, the electrodes 160 are directed so as to face away from the framework 120.

With specific reference to FIGs. 2 and FIG. 3E, each flexible circuit 110, 150 includes a plurality of electrodes 160A (on first flexible circuit 110), 160B (on second flexible circuit 150), a base 112 comprising a soldering pad region disposed at a proximal end thereof, a plurality 114A-G of branch segments 114 extending from the base 112, a plurality of voids 113A-113J defined between the branch segments 108A-108C, and a connecting bridge 116.

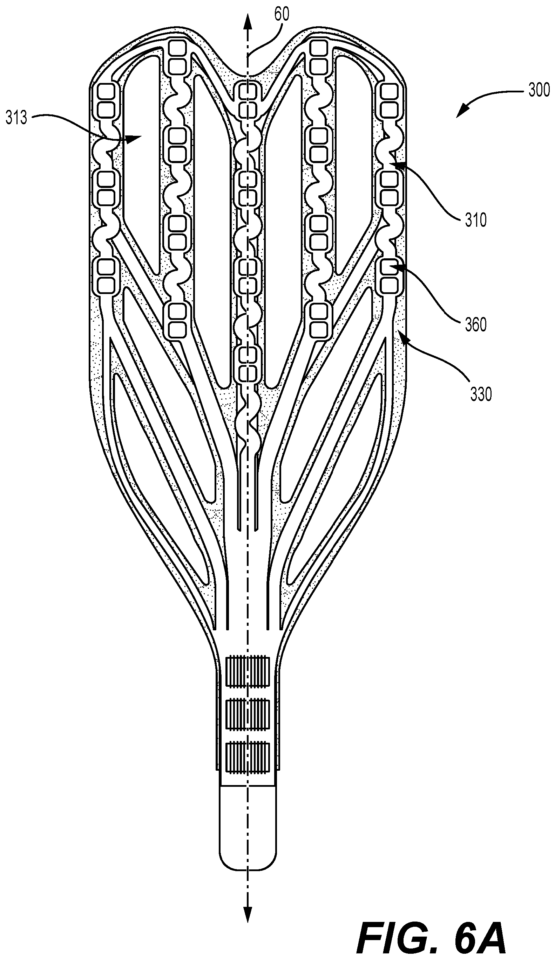

A central branch segment 114D of the branch segments 114 extends along the longitudinal axis 60, while outer branch segments, 114A, 114B, 114C, 114E, 114F, 114G extend from the base 112 away from the longitudinal axis 60 and then generally parallel thereto. The electrodes 160A can be disposed along the branch segments 114B-114F such that they are aligned relative to electrodes 160A on adjacent branch segments 114B-114F along the longitudinal axis 60. In other examples (e.g., FIG. 6A), the electrodes 160A can be unaligned (i.e., staggered) relative to electrodes 160A on adjacent branches 114B-114F in a direction transverse to the longitudinal axis 60 such that they are arranged in an alternatingly aligned pattern from branch segment to branch segment.

Additionally, the branch segments include connecting outer segments 114A, 114G that do not include electrodes 160A. Rather, these segments 114A, 114G aid in defining the shape of the first end effector 100 and provide reinforcement/protection to the segments of the flexible circuit 110 that carries electrical traces and/or electrodes 160.

As seen in FIG. 3E, the configuration of the different layers of the first end effector 100 (e.g., flexible circuits 110, 150, position sensor 140, insulative material 130, and framework 120) results in a plurality of voids 113A-113J being defined between respective sections of the first end effector 100. Specifically, voids 113A-113J (i.e., an area not covered by any material) are defined between the respective branch segments 114A-114G of the flexible circuit 110. Moreover, these voids are further subdivided in regions where the supports of the framework 120 run (e.g., first support 126A results in two voids 113B, 113C between outer branch segments 114B, 114C). This reduction of material aids in facilitating the collapsing of the first end effector 100 into the sheath and/or insertion tool.

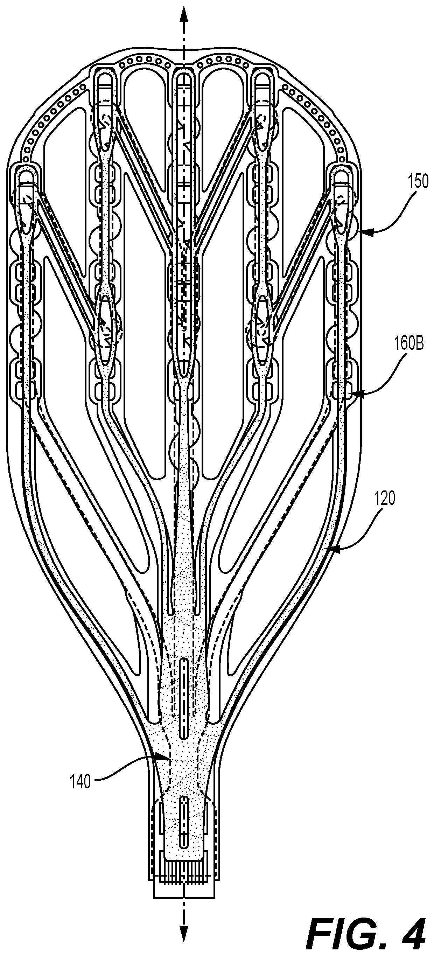

As shown in FIG. 4, the eyelets 124A3, 124B4, 124B6, 124D4, 124D6, 124E3, in addition to aligning with the curved connecting portions 144A3, 144A5, 144A7, 144B7, 144B5, 144B3 of the position sensor 140, also approximately aligns with many of electrodes 160A, thereby providing additional support thereto and more evenly distributing forces incurred by the flexible circuit 110.

It is noted that not all of the electrodes 160A on the first end effector 110 described herein need be exposed through the insulative material 130 as these non-exposed electrodes can be used to sense far-field signals for noise reduction proximate the tissue contacting electrodes. Similarly, far-field signals including noise or artifacts can be reduced or canceled out for the overall end effector with a reference electrode that is not in contact with tissues and only with blood.

Turning now to FIGs. 5A-5B, a portion of a second exemplary end effector 200 is shown (similar to the view of FIG. 4) that incorporates similar concepts as those discussed above with respect to the first end effector 100, such as, eyelets and supports in the framework 220 and voids 213A-L in the overall second end effector 200.

Making specific reference to FIG. 5A, the framework 220 of the second end effector 200 includes a base 222, a plurality of spines 224A-E extending therefrom along the longitudinal axis 60, and a plurality of supports 226A-D connecting adjacent spines 224A-E. In some examples, there are five cantilevered spines (i.e., a first cantilevered spine 224A, a second cantilevered spine 224B, a third, central cantilevered spine 224C, a fourth cantilevered spine 124D, and a fifth cantilevered spine 124E); however, other numbers of cantilevered spines can be used without departing from the spirit and scope of the present disclosure. Moreover, it is noted that, like the previously described example the spines 224A-E are cantilevered because their distalmost tips/ends are unsupported/unfixed. In some examples, the spines 224A-E can be supported at their distalmost tips/ends (i.e., they are not cantilevered).

The first cantilevered spine 224A (also referred to herein as the "first spine") extends from the base 222 along the longitudinal axis 60 to an unsupported distalmost end. The first cantilevered spine 224A includes (1) a first segment 224A1 initially extending at an oblique angle, relative to the longitudinal axis 60, from the base 222 and (2) a second segment extending from the first segment 224A1 parallel to the longitudinal axis 60 and forming a terminal portion 224A2 including (i) an elongated eyelet 224A2 and (ii) the unsupported distalmost end of the first cantilevered spine 224A. The elongated eyelet 224A2 aids in supporting other sub-components of the second end effector 200 and is discussed in greater detail below.

The second cantilevered spine 224B (also referred to herein as the "second spine") extends from the base 222 along the longitudinal axis 60 to an unsupported distalmost end. The second cantilevered spine 224B includes (1) a first segment 224B1 initially extending at an oblique angle, relative to the longitudinal axis 60, from the base 222 and (2) a second segment extending from the first segment 224B1 parallel to the longitudinal axis 60, the second segment forming a terminal portion 224B2 including an (i) elongated eyelet 224B2 and (ii) the unsupported distalmost end of the second cantilevered spine 224B. The elongated eyelet 224B2 aids in supporting other sub-components of the second end effector 200 and is discussed in greater detail below.

The third cantilevered spine 224C (also referred to herein as the "third spine") is centrally located relative two the first/second cantilevered spines 224A, 224B and the fourth/fifth cantilevered spines 224D, 224E. It extends from the base 222 to an unsupported distalmost end. The third cantilevered spine 224C includes (1) a first segment 224C1 extending generally parallel to or co-axial with the longitudinal axis 60 from the base 222 and (2) a second segment forming a terminal portion 224C2 including (i) an elongated eyelet 224C2 that has an elongated shape and (ii) the unsupported distalmost end.

The fourth cantilevered spine 224D (also referred to herein as the "fourth spine") extends from the base 222 along the longitudinal axis 60 to an unsupported distalmost end and is generally symmetrical with the second cantilevered spine 224B relative to the longitudinal axis 60. The fourth cantilevered spine 224D includes (1) a first segment 224D1 initially extending at an oblique angle, relative to the longitudinal axis 60, from the base 222 and (2) a second segment extending from the first segment 224D1 parallel to the longitudinal axis 60, the second segment forming a terminal portion 224D2 including an (i) elongated eyelet 224D2 and (ii) the unsupported distalmost end of the fourth cantilevered spine 224D. The elongated eyelet 224D2 aids in supporting other sub-components of the second end effector 200 and is discussed in greater detail below.

The fifth cantilevered spine 224E (also referred to herein as the "fifth spine") extends from the base 222 along the longitudinal axis 60 to an unsupported distalmost end and is generally symmetrical with the first cantilevered spine 224A relative to the longitudinal axis 60. The fifth cantilevered spine 224E includes (1) a first segment 224E1 initially extending at an oblique angle, relative to the longitudinal axis 60, from the base 222 and (2) a second segment extending from the first segment 224E1 parallel to the longitudinal axis 60 and forming a terminal portion 224E2 including (i) an elongated eyelet 224E2 and (ii) the unsupported distalmost end of the fifth cantilevered spine 224E. The elongated eyelet 224E2 aids in supporting other sub-components of the second end effector 200 and is discussed in greater detail below.

As mentioned above, a plurality of supports 226A-D interconnect respective adjacent spines 224A-E. More specifically, the supports include a first support 226A, a second support 226B, a third support 226C, and a fourth support 226D. These supports 226A-D extend at an oblique (i.e., non-perpendicular) angle relative to the longitudinal axis 60 in the distal direction from one spine to another.

The first support 226A connects the eyelet 224A2 (i.e., a terminal portion) of the first spine 224A with a connecting region (i.e., an intermediate portion) between the first segment 224B1 and the eyelet 224B2 of the second spine 224B. The second support 226A connects the eyelet 224B2 (i.e., the terminal portion) of the second spine 224B with the elongated eyelet 224C2, more proximal to the base 222 (and thus, at an intermediate portion), of the third spine 124C. The third support 226C is mirrored symmetrically with the second support 226B and connects the eyelet 224C2 of the third spine 224C with the eyelet 224D2 of the fourth spine 224D. The fourth support 226D is mirrored symmetrically with the first support 226A and connects a connecting region of the fourth spine 224D with the eyelet 224E2 of the fifth spine 124E.

As seen particularly in FIG. 5A, the first and second supports 226A, 226B extend in an approximately parallel manner. Additionally, a proximal portion of the first segment 224A1 of the first spine 224A and the first segment 224B1 of the second spine 224B are substantially parallel with the first and second supports 226A, 226B. Likewise, the third and fourth supports 226C, 226D are substantially parallel to one another, with a proximal portion of the first segment 224E1 of the fifth spine 224E and the first segment 224D1 of the fourth spine 224D also being parallel thereto.

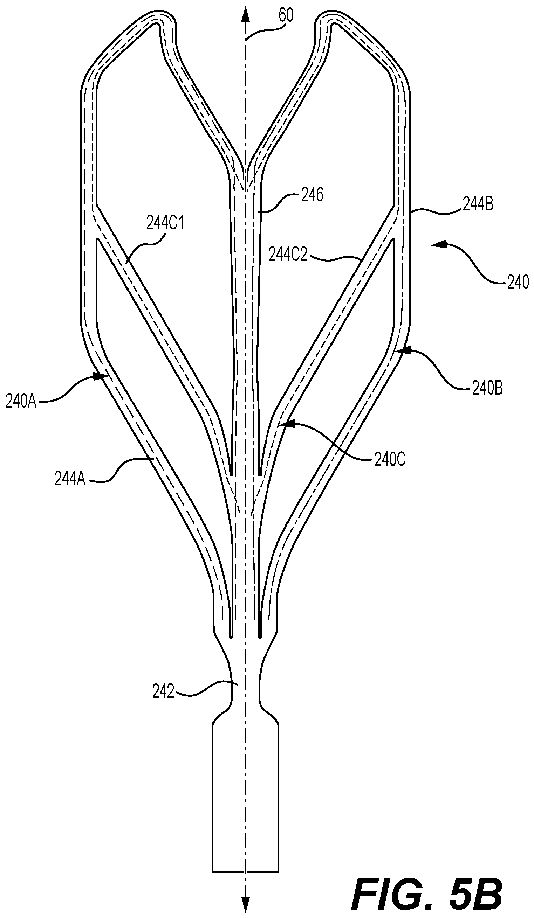

Making specific reference to FIGs. 5A and 5B, a position sensor 240 can be provided that is sandwiched between the second flexible circuit 250 (or a first flexible circuit) and the framework 220, generally parallel thereto, and spaced therefrom along the vertical axis 62. In this example, the position sensor 240 is configured as a flexible circuit (e.g., similar to flexible circuit 250, but without electrodes formed thereon) that has one or more loops 240A, 240B, 240C with traces that each, when subjected to a magnetic field, induce a current indicative of a position of the second end effector 200.

Moreover, as particularly shown in FIG. 5A, a plurality of segments of the position sensor 240 (which is shown with stippling for clarity) run along portions of the framework 220, such as the eyelets, providing support to the position sensor. Similarly, the supports 226A-D extend parallel to and aligned with certain segments of the position sensor 240.

Referring specifically to FIG. 5B, the position sensor 240 includes a base 242, a first side section 244A laterally offset from the longitudinal axis 60, a second side section 244B laterally offset from the longitudinal axis 60, a first connecting segment 244C1, a second connecting segment 244C2, and a center segment 246 running along the longitudinal axis 60.

The first side section 244A extends from the base 242 obliquely relative to the longitudinal axis 60 and loops around to connect with a distal end of the center segment 246. The second side section 244B also extends from the base 242 obliquely relative to the longitudinal axis 60 and loops around to connect with the distal end of the center segment 246. The first connecting segment 244C1 connects to a distal end of the base 242 and an intermediate segment of the first side section 244A. The second connecting segment 244C2 connects to the distal end of the base 242 and an intermediate segment of the second side section 244A.

As seen in FIG. 5B and denoted by the phantom lines, the position sensor 240 further includes a pair of side location sensing loops 240A (long dash phantom lines), 240B (unevenly sized phantom lines) disposed generally symmetrical about the longitudinal axis 60 that run along the aforementioned side sections 244A, 244B and center segment 246. The center loop 240C partially overlaps with both side loops 240A, 240B and runs along both connecting segments 244C1, 244C2 as well as portions of both the first side section 244A and the second side section 244B. In general, each loop 240A, 240B, 240C extends in a loop from a proximal portion of the framework 220, to a distal portion of the framework 220, and back to the proximal portion of the framework 220 along the longitudinal axis 60. The first side loop 240A extends along the first side section 244A and the center segment 246, while the second side loop 240B extends along the second side section 244B and the center segment 246.

The insulative material 230 and flexible circuits (e.g., second flexible circuit 250) can be configured similarly to the first end effector 100, and are therefore not described in comprehensive detail here, since an understanding of this example can be derived from the foregoing disclosure. It is noted that, as seen best in FIG. 5A, the electrodes 260 disposed on branches of the flexible circuit 250 run along/align with the eyelets of the framework 220, thereby providing additional support to the electrodes 260. Moreover, similar to the configuration shown in FIG. 3D, the electrodes 260 can align with electrodes 260 on adjacent branched.

As seen in FIG. 5A the configuration of the different layers of the second end effector 200 (e.g., flexible circuit 250, position sensor 240, insulative material 230, and framework 220) results in a plurality of voids 213A-213J being defined between respective sections of the second end effector 200. Specifically, voids 213A-213J (i.e., an area not covered by any material) are defined between the respective branch segments of the flexible circuit 250. Moreover, these voids are further subdivided in regions where the supports of the framework 220 run (e.g., first support 226A results in two voids 213B, 213C between the two left outer branch segments of the flexible circuit 250). This reduction of material aids in facilitating the collapsing of the second end effector 200 into the sheath and/or insertion tool.

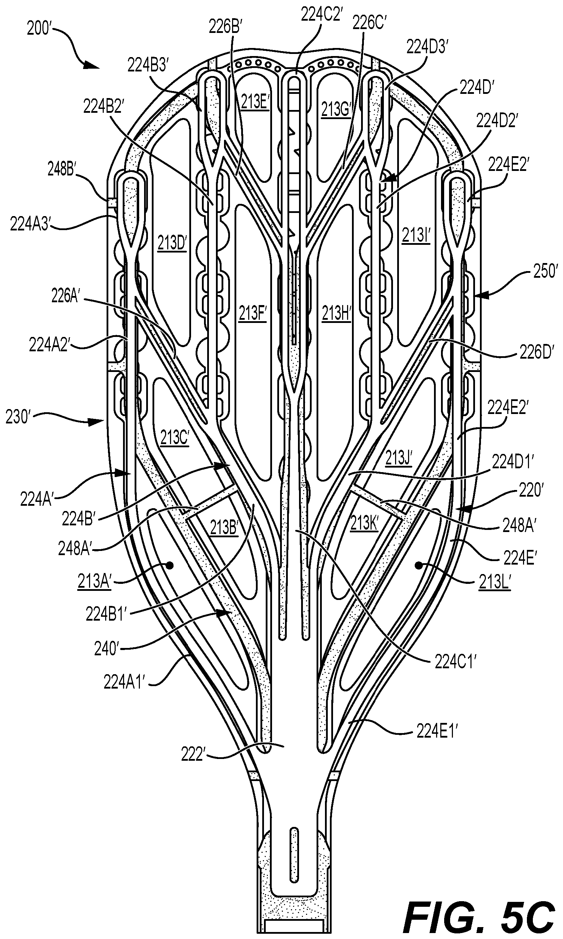

Turning now FIG. 5C, a portion of a modified configuration of the second exemplary end effector 200' is shown (similar to the view of FIG. 5A) that incorporates similar concepts as those discussed above with respect to the first end effector 100 and the second end effector 200, such as, eyelets and supports in the framework 220 and voids 213A-L in the overall second end effector 200.

Making specific reference to FIG. 5C, the framework 220' of the modified second end effector 200 includes a base 222', a plurality of spines 224A'-E' extending therefrom along the longitudinal axis 60, and a plurality of supports 226A'-D' connecting adjacent spines 224A'-E'. In some examples, there are five cantilevered spines (i.e., a first cantilevered spine 224A', a second cantilevered spine 224B', a third, central cantilevered spine 224C', a fourth cantilevered spine 124D', and a fifth cantilevered spine 124E'); however, other numbers of cantilevered spines can be used without departing from the spirit and scope of the present disclosure. Moreover, it is noted that, like the previously described example the spines 224A'-E' are cantilevered because their distalmost tips/ends are unsupported/unfixed. In some examples, the spines 224A'-E' can be supported at their distalmost tips/ends (i.e., they are not cantilevered).

The first cantilevered spine 224A' (also referred to herein as the "first spine") extends from the base 222' along the longitudinal axis 60 to an unsupported distalmost end. The first cantilevered spine 224A' includes (1) a first segment 224A1' initially extending at an oblique angle, relative to the longitudinal axis 60, from the base 222' and (2) a second segment 224A2' extending from the first segment 224A1' parallel to the longitudinal axis 60 to a terminal portion 224A3' including (i) an eyelet 224A3 and (ii) the unsupported distalmost end of the first cantilevered spine 224A'. The eyelet 224A3' aids in supporting other sub-components of the modified second end effector 200' and is discussed in greater detail below.

The second cantilevered spine 224B' (also referred to herein as the "second spine") extends from the base 222' along the longitudinal axis 60 to an unsupported distalmost end. The second cantilevered spine 224B' includes (1) a first segment 224B1' initially extending at an oblique angle, relative to the longitudinal axis 60, from the base 222' and (2) a second segment 224B2' extending from the first segment 224B1' parallel to the longitudinal axis 60 to a terminal portion 224B3' including an (i) eyelet 224B3' and (ii) the unsupported distalmost end of the second cantilevered spine 224B'. The eyelet 224B3' aids in supporting other sub-components of the modified second end effector 200' and is discussed in greater detail below.

The third cantilevered spine 224C' (also referred to herein as the "third spine") is centrally located relative two the first/second cantilevered spines 224A', 224B' and the fourth/fifth cantilevered spines 224D', 224E'. It extends from the base 222' to an unsupported distalmost end. The third cantilevered spine 224C' includes (1) a first segment 224C1' extending generally parallel to or co-axial with the longitudinal axis 60 from the base 222' and (2) a second segment forming a terminal portion 224C2' including (i) an elongated eyelet 224C2' that has an elongated shape and (ii) the unsupported distalmost end.

The fourth cantilevered spine 224D' (also referred to herein as the "fourth spine") extends from the base 222' along the longitudinal axis 60 to an unsupported distalmost end and is generally symmetrical with the second cantilevered spine 224B' relative to the longitudinal axis 60. The fourth cantilevered spine 224D' includes (1) a first segment 224D1' initially extending at an oblique angle, relative to the longitudinal axis 60, from the base 222' and (2) a second segment 224D2' extending from the first segment 224D1' parallel to the longitudinal axis 60 to a terminal portion 224D3' including an (i) eyelet 224D3' and (ii) the unsupported distalmost end of the fourth cantilevered spine 224D'. The eyelet 224D3' aids in supporting other sub-components of the modified second end effector 200' and is discussed in greater detail below.

The fifth cantilevered spine 224E' (also referred to herein as the "fifth spine") extends from the base 222' along the longitudinal axis 60 to an unsupported distalmost end and is generally symmetrical with the first cantilevered spine 224A' relative to the longitudinal axis 60. The fifth cantilevered spine 224E' includes (1) a first segment 224E1' initially extending at an oblique angle, relative to the longitudinal axis 60, from the base 222' and (2) a second segment 224E2' extending from the first segment 224E1' parallel to the longitudinal axis 60 to a terminal portion 224E3' including (i) an eyelet 224E3' and (ii) the unsupported distalmost end of the fifth cantilevered spine 224E'. The eyelet 224A3' aids in supporting other sub-components of the modified second end effector 200' and is discussed in greater detail below.

As mentioned above, a plurality of supports 226A'-D' interconnect respective adjacent spines 224A'-E'. More specifically, the supports include a first support 226A', a second support 226B', a third support 226C', and a fourth support 226D'. These supports 226A'-D' extend at an oblique (i.e., non-perpendicular) angle relative to the longitudinal axis 60 in the distal direction from one spine to another.

The first support 226A' connects the second segment 224A2' of the first spine 224A' with a connecting region (i.e., an intermediate portion) between the first segment 224B1' and the second segment 224B2' of the second spine 224B'. The second support 226A' connects the eyelet 224B2' (i.e., the terminal portion) of the second spine 224B' with the elongated eyelet 224C2', more proximal to the base 222' (and thus, at an intermediate portion), of the third spine 124C'. The third support 226C' is mirrored symmetrically with the second support 226B' and connects the eyelet 224C2' of the third spine 224C with the eyelet 224D2' of the fourth spine 224D'. The fourth support 226D' is mirrored symmetrically with the first support 226A' and connects a connecting region of the fourth spine 224D' with the second segment 224E2' of the fifth spine 124E'.

The first and second supports 226A', 226B' extend in an approximately parallel manner. Additionally, a proximal portion of the first segment 224A1' of the first spine 224A' and the first segment 224B1' of the second spine 224B' are substantially parallel with the first and second supports 226A', 226B'. Likewise, the third and fourth supports 226C', 226D' are substantially parallel to one another, with a proximal portion of the first segment 224E1' of the fifth spine 224E' and the first segment 224D1' of the fourth spine 224D' also being parallel thereto.

Making continued reference to FIG. 5C, a position sensor 240' can be provided that is sandwiched between the second flexible circuit 250' (or a first flexible circuit) and the framework 220', generally parallel thereto, and spaced therefrom along the vertical axis 62. In this example, the position sensor 240' is configured as a flexible circuit (e.g., similar to flexible circuit 250, but without electrodes formed thereon) that has one or more with traces that each, when subjected to a magnetic field, induce a current indicative of a position of the modified second end effector 200'. The position sensor 240' can be configured similarly or identically to the previously described position sensor 240 of FIG. 5B. Additional features of the modified position sensor 240' include branches 248A' for supporting the respective loops and tabs 248B' that aid in adhering the position sensor 240A' to the insulative material 230'.

Moreover, as particularly shown in FIG. 5C, a plurality of segments of the position sensor 240 (which is shown with stippling for clarity) run along portions of the framework 220', such as the eyelets, providing support to the position sensor. Similarly, the supports 226A'-D' extend parallel to and aligned with certain segments of the position sensor 240'.

As seen in FIG. 5C the configuration of the different layers of the modified second end effector 200' (e.g., flexible circuit 250', position sensor 240', insulative material 230, and framework 220) results in a plurality of voids 213A'-213L' being defined between respective sections of the second end effector 200. Specifically, voids 213A'-213J' (i.e., an area not covered by any material) are defined between the respective branch segments of the flexible circuit 250'. Moreover, these voids are further subdivided in regions where the branches of the position sensor 240' run (e.g., left branch 248A' results in two voids 213B', 213C' between the two left outer branch segments of the flexible circuit 250'). This reduction of material aids in facilitating the collapsing of the modified second end effector 200' into the sheath and/or insertion tool.

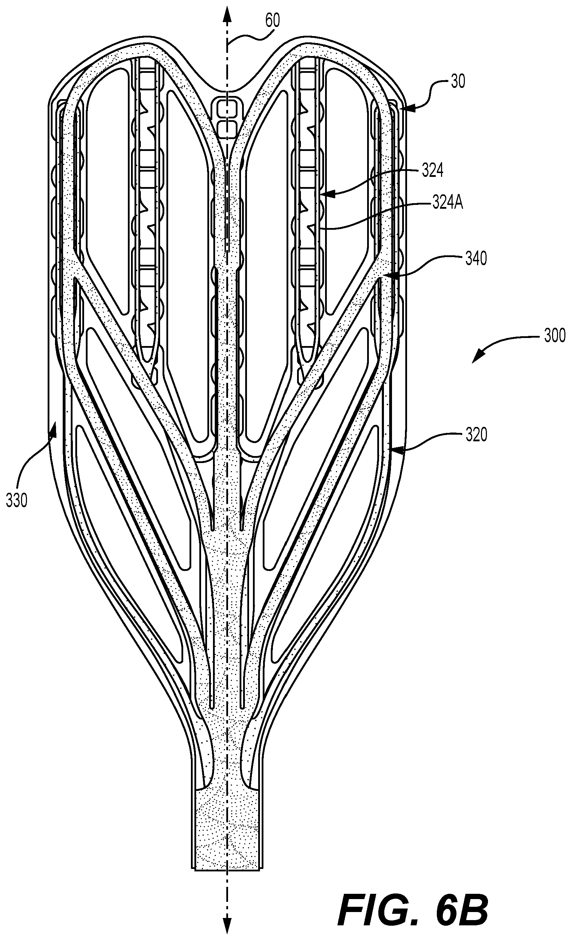

FIGs. 6A-6C depict yet another, third exemplary end effector 300 that incorporates similar features as that of ones previously discussed. For that reason, all the features of the third end effector 300 are not described in comprehensive details, since an understanding of this example can be derived from the foregoing disclosure. For example, similar to the other examples, the third end effector 300 includes one or more flexible circuits 310 with electrodes 360, a framework 320, an insulative material 330, and a position sensor 340. In this example, as best seen in FIG. 6A, the electrodes 360 are unaligned/staggered. As shown partially in FIG. 6B, the framework 320 employs a plurality of spines 324, such as cantilevered spine 324A, that include elongated eyelets for supporting the branches/electrodes 360 of the flexible circuit 310.

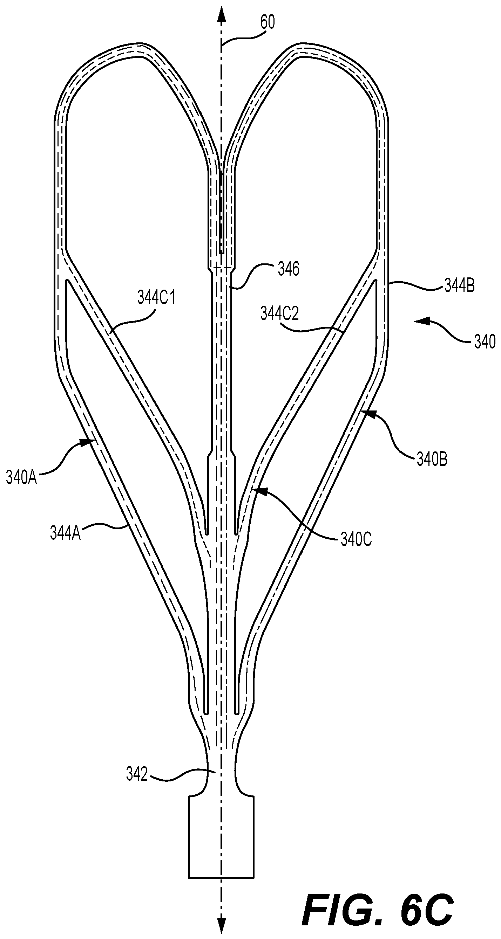

FIG. 6C depicts a position sensor 340 that can be provided that is sandwiched between one of the flexible circuits and the framework 320, generally parallel thereto, and spaced therefrom along the vertical axis 62. In this example, the position sensor 340 is configured as a flexible circuit that has three loops 340A, 340B, 340C (similar to the example shown in FIG. 5B) with traces that each, when subjected to a magnetic field, induce a current indicative of a position of the second end effector 200.

As particularly shown in FIG. 6B, a plurality of segments of the position sensor 340 (which is shown with stippling for clarity) run along portions of the framework 320, such as the eyelets, providing support to the position sensor 340. Similarly, supports (not shown) of the framework 320 extend parallel to and aligned with certain segments of the position sensor 340.

Referring specifically to FIG. 6C, the position sensor 340 (which is similar in structure and function as that of position sensor 240) includes a base 342, a first side section 344A laterally offset from the longitudinal axis 60, a second side section 344B laterally offset from the longitudinal axis 60, a first connecting segment 344C1, a second connecting segment 344C2, a center segment 346 running along the longitudinal axis 60.

The first side section 344A extends from the base 342 obliquely relative to the longitudinal axis 60 and loops around to connect with a distal end of the center segment 346. The second side section 344B also extends from the base 342 obliquely relative to the longitudinal axis 60 and loops around to connect with the distal end of the center segment 346. The first connecting segment 344C1 connects to a distal end of the base 342 and an intermediate segment of the first side section 344A. The second connecting segment 344C2 connects to the distal end of the base 342 and an intermediate segment of the second side section 344A.

As seen in FIG. 6C and denoted by the phantom lines, the position sensor 340 further includes a pair of side location sensing loops 340A (long dash phantom lines), 340B (unevenly sized phantom lines) disposed generally symmetrical about the longitudinal axis 60 that run along the aforementioned side sections 344A, 344B and center segment 346. The center loop 340C partially overlaps with both side loops 340A, 340B and runs along both connecting segments 344C1, 344C2 as well as portions of both the first side section 344A and the second side section 344B. In general, each loop 340A, 340B, 340C extends in a loop from a proximal portion of the framework 320, to a distal portion of the framework 320, and back to the proximal portion of the framework 320 along the longitudinal axis 60. The first side loop 340A extends along the first side section 344A and the center segment 346, while the second side loop 340B extends along the second side section 344B and the center segment 346.

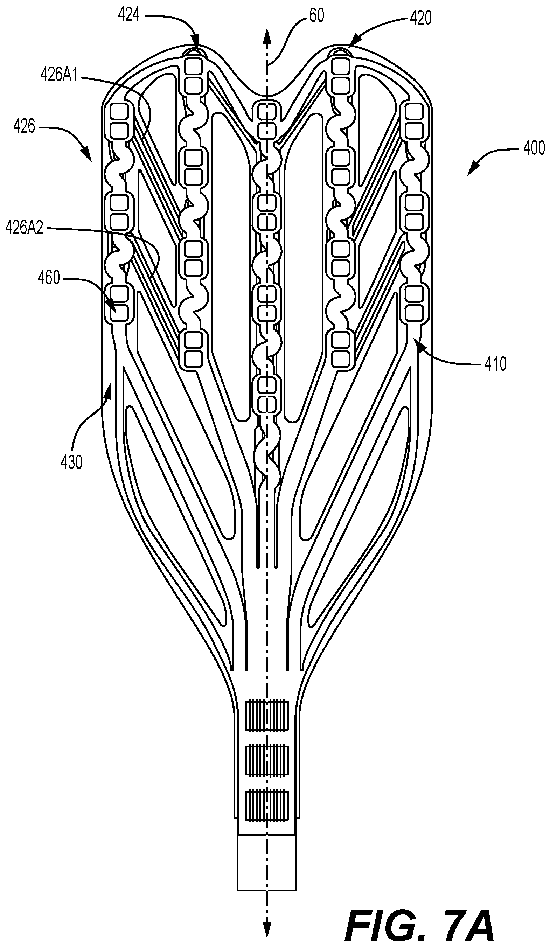

FIGs. 7A-7C depict yet another, fourth exemplary end effector 400 that incorporates similar features as that of ones previously discussed. For that reason, all the features of the fourth end effector 400 are not described in comprehensive details, since an understanding of this example can be derived from the foregoing disclosure. For example, similar to the other examples, the fourth end effector 300 includes one or more flexible circuits 410 with electrodes 460, a framework 420, an insulative material 430, and a position sensor 440. In this example, as best seen in FIG. 7A, the electrodes 460 are unaligned/staggered.

The framework 420 employs a plurality of cantilevered spines 424 that each include one or more eyelets for supporting the branches/electrodes 460 of the flexible circuit 410. In this example, one or more of the supports 426 that connects adjacent spines can be configured as a pair of supports 426A, 426B that extend approximately parallel to one another and each have a pair of legs (like in the example explained with respect to FIG. 3A).

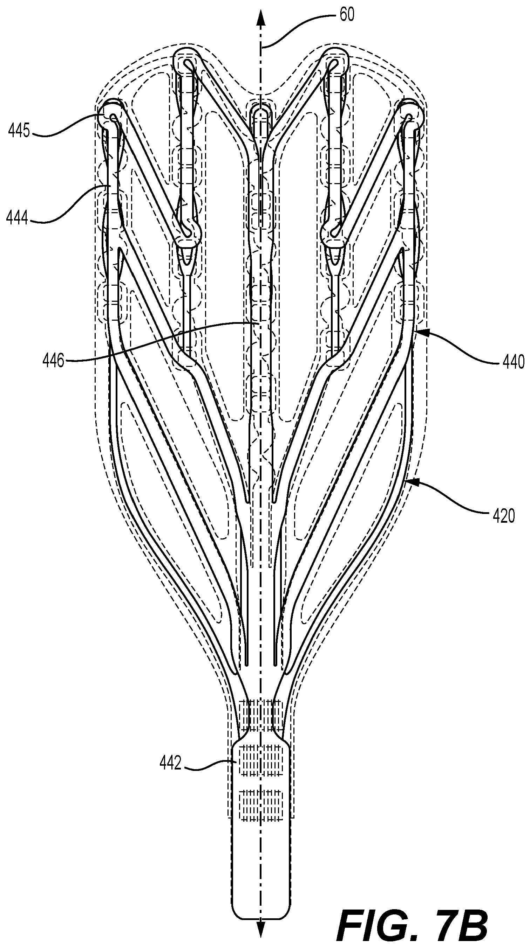

FIG. 7B depicts a position sensor 440 that is supported by the framework 420, generally parallel thereto, and spaced therefrom along the vertical axis 62. In FIG. 7B, other subcomponents of the end effector 400 are shown in phantom lines to illustrate a relative positioning of the position sensor 440. In this example, the position sensor 440 is configured as a flexible circuit that has three loops 440A, 440B, 440C (similar to the example shown in FIG. 6C) with traces that each, when subjected to a magnetic field, induce a current indicative of a position of the second end effector 200.

As particularly shown in FIG. 7B, a plurality of segments of the position sensor 440 run along portions of the framework 420, such as the eyelets, providing support to the position sensor 440. Similarly, supports 426A1, 426A2 of the framework 420 extend parallel to and aligned with certain segments of the position sensor 440 (as can be seen by comparing FIGs. 7A and 7B).

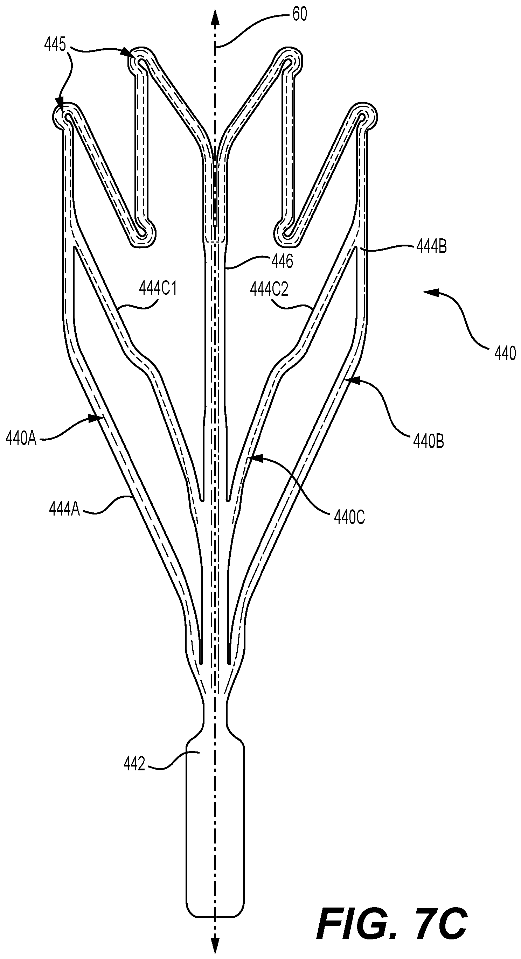

Referring specifically to FIG. 7C, the position sensor 440 (which is similar in structure and function as that of position sensors 240, 340) includes a base 442, a first side section 444A laterally offset from the longitudinal axis 60, a second side section 444B laterally offset from the longitudinal axis 60, a first connecting segment 444C1, a second connecting segment 444C2, a center segment 446 running along the longitudinal axis 60, and one or more curved connecting portions 445 connecting segments of either the first side section 444A or the second side section 444B.

In greater detail, the first side section 444A extends from the base 442 obliquely relative to the longitudinal axis 60 and loops around, including three redirects with the curved connecting portions 445, to connect with a distal end of the center segment 446. The second side section 444B also extends from the base 442 obliquely relative to the longitudinal axis 60 and loops around, including three redirects with the curved connecting portions 445, to connect with the distal end of the center segment 346. The first connecting segment 444C1 connects to a distal end of the base 442 and an intermediate segment of the first side section 444A. The second connecting segment 444C2 connects to the distal end of the base 442 and an intermediate segment of the second side section 444A.

As seen in FIG. 7C and denoted by the phantom lines, the position sensor 440 further includes a pair of side location sensing loops 440A (long dash phantom lines), 440B (unevenly sized phantom lines) disposed generally symmetrical about the longitudinal axis 60 that run along the aforementioned side sections 444A, 444B and center segment 446. A center loop 440C partially overlaps with both side loops 440A, 440B and runs along both connecting segments 444C1, 444C2 as well as portions of both the first side section 444A and the second side section 444B. In general, each loop 440A, 440B, 440C extends in a loop from a proximal portion of the framework 420, to a distal portion of the framework 420, and back to the proximal portion of the framework 420 along the longitudinal axis 60. The first side loop 440A extends along the first side section 444A and the center segment 446, while the second side loop 440B extends along the second side section 444B and the center segment 446.

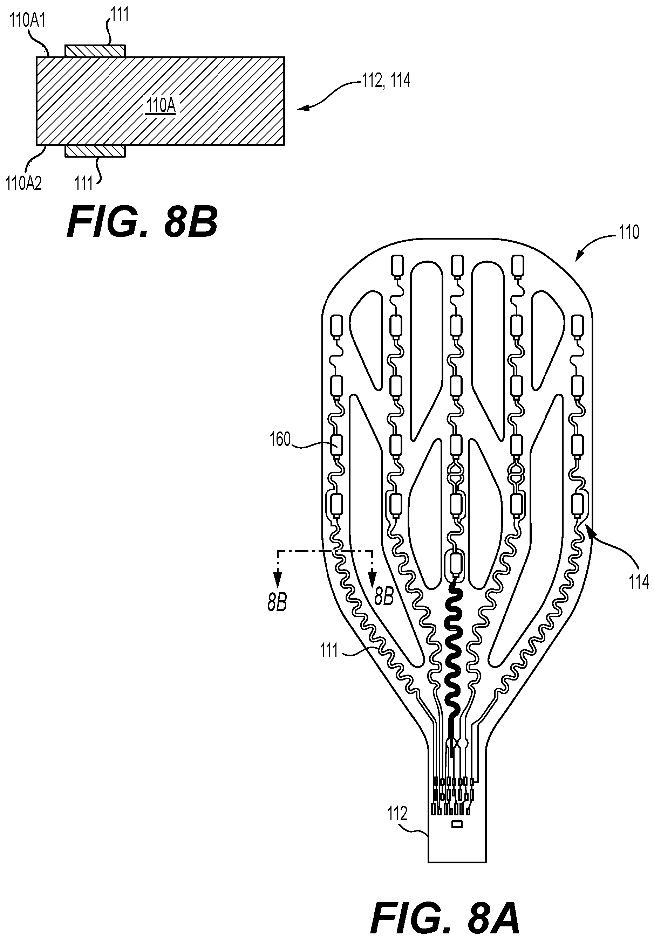

Turning now to FIGs. 8A-11, another aspect of the present disclosure is described in the following. Making reference to FIGs. 8A and 8B, the end effectors (100, 200, 300, 100') described herein can use a stretchable and/or soft polymer, such as TPU, as the base substrate layer 110A (e.g., as part of the base 112 and branches 114 of the flexible circuit 110), with conductive material 111 attached directly to the soft polymer substrate. Other examples of stretchable and/or soft polymers appropriate for use as the base substrate layer 110A include, but are not limited to, other thermoplastics such as thermoplastic elastomer (TPE), thermoplastic copolyester (TPC), and thermoplastic vulcanizate (TPV), silicone, BEYOLEX^{™}, and the like. As used herein, the term "stretchable" refers to a material that is capable of elastically deforming. In other words, in some examples, the base substrate layer and/or the flexible circuit can be polyimide-less and LCP-less, which can improve flexibility and/or stretchability of the overall end effector.

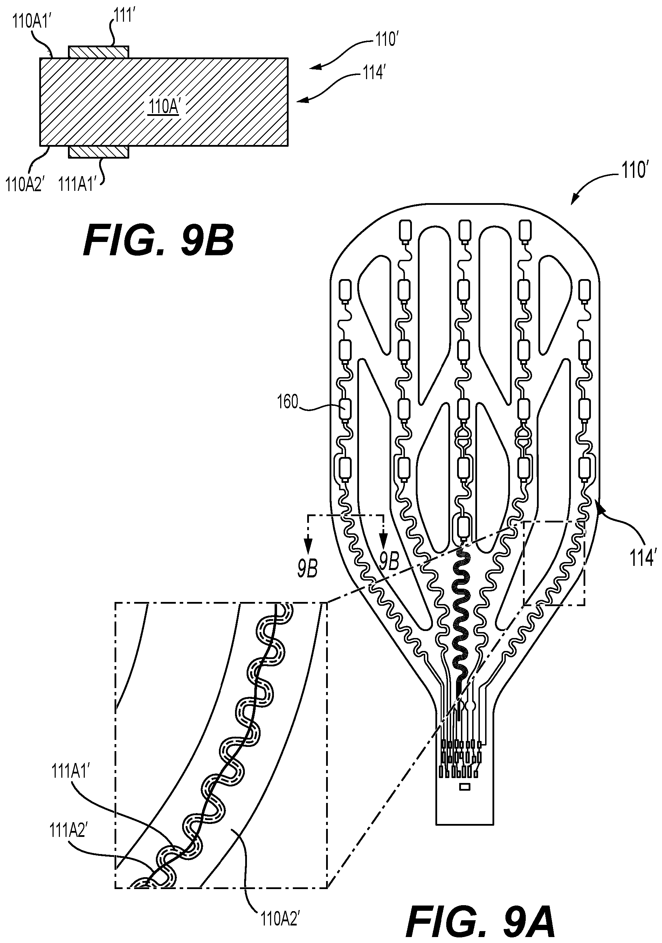

On flexible circuits 110, as seen in FIGs. 8A-8B, the conductive material 111 can be routed on (1) a first, upper surface 110A1, (2) a second, lower surface 110A2, or (3) both surfaces 110A1, 110A2 and is connected to electrodes 160 that are connected to the one of the substrate surfaces/layers. As will be appreciated by those skilled in the art, by using a soft polymer substrate layer 110A, the conductive material 111 (e.g., traces) are, therefore, a primary limiting factor for strain capacity during bending/torsional load applied to the end effector. For that reason, the presently disclosed technology includes flexible circuits 110' that have one or more strain reduction features connected to the substrate layer 110A' (formed from a soft polymer, as discussed above). These strain reduction features are described in greater detail in FIGs. 9A-12.

Making reference to FIGs. 9A-9B, a flexible circuit 110' is shown that includes a plurality of branches 114', along which one or more traces 111' are routed along and connect to electrodes 160. To protect these electrode traces 111', this example includes one or more sacrificial traces 111A1', 111A2' that are connected to one of the surfaces 110A1', 110A2' of the substrate layer 110A' (e.g., a surface 110A2' of the substrate layer 110A' opposite that of the electrode trace 111'). As discussed above, the substrate layer 110A' can be formed from a stretchable and/or soft polymer, such as TPU, such that the substrate layer 110A' is flexible and stretchable in three dimensions. Electrode trace 111' can have an undulating serpentine pattern, such as the one denoted by the dashed line in the detail view forming a part of FIG. 9A. To reduce the strain on this trace 111', the sacrificial trace 111A2' can have a shallower serpentine (i.e., lower frequency of undulations/oscillations) or run substantially linearly/straight. Alternatively, the sacrificial trace 111A1' can have the same serpentine/undulating pattern as that of the electrode trace 111'. These sacrificial traces 111A1', 111A2' are solely used for mechanical functionality by limiting the strain on the other electrical traces 111'. Exemplary materials for the sacrificial traces 111A1', 111A2' include, but are not limited to, copper, gold, Nitinol, platinum, and combinations thereof.

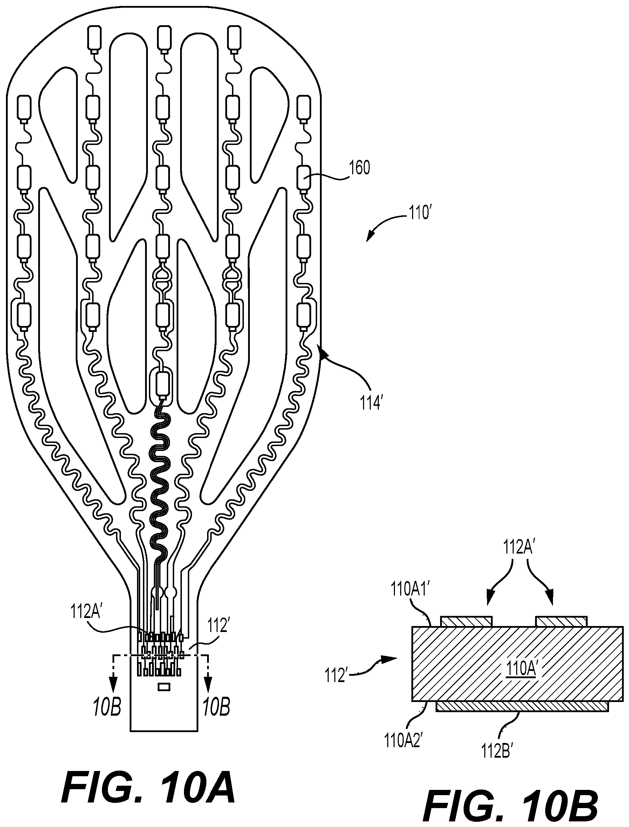

Turning now to FIGs. 10A-10B, a strain reduction feature for the base 112' that includes a termination/soldering pad area 112A' is shown. In this region of the substrate layer 110A', opposite the soldering pad region 112A', a heat sink 112B' can be connected to the substrate layer 110A' that dissipates heat from the soldering pad region 112A' in use. The heat sink 112B' can be, for example, configured as a polyimide layer with a conductive material (e.g., gold, copper, or the like) connected to the second surface 110A2' while the soldering pad region 112A' is on the first surface 110A1' of the substrate layer 110A'.

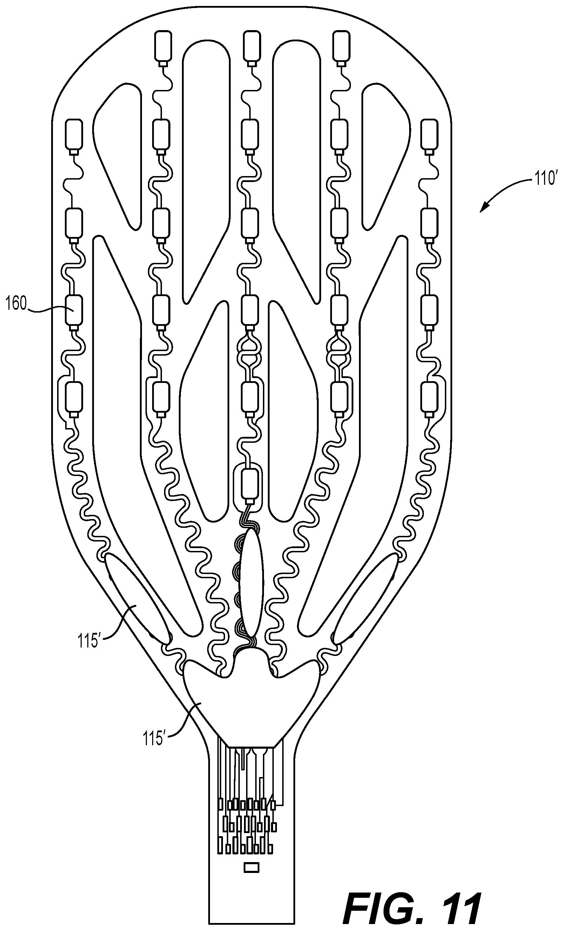

Turning to FIG. 11, another strain reduction feature is shown that is embodied as one or more localized stiffeners 115'. The stiffeners 115' can be added to the top or bottom substrate layers 110A1', 110A2', in a region in which trace(s) 111' run, and can take various forms, such as a metal place or a polyimide pad.

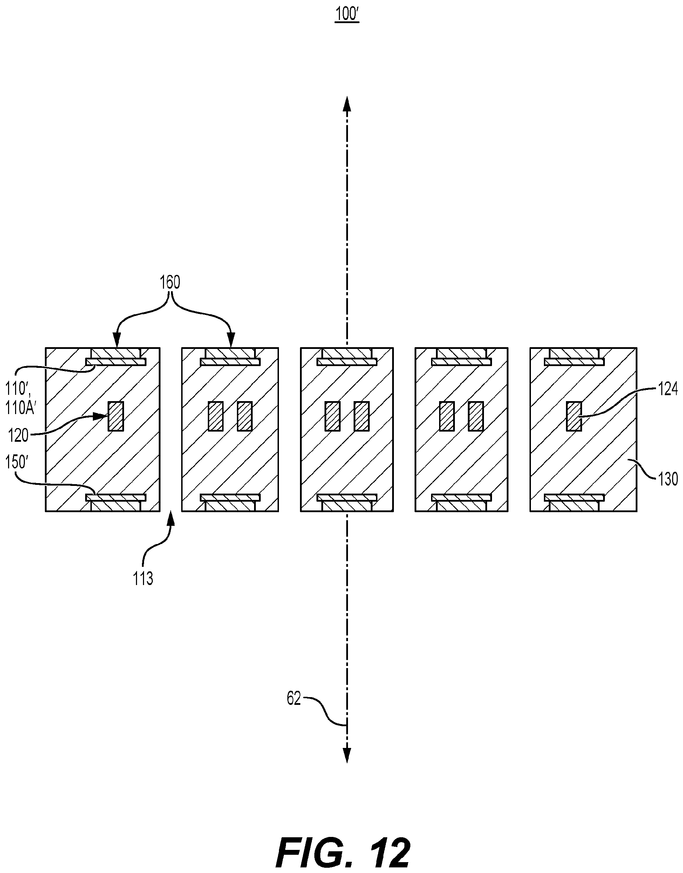

FIG. 12 depicts an exemplary cross-sectional view, cut perpendicular to the longitudinal axis 60 of an end effector 100' of a catheter that includes the previously described flexible circuit 110' of any one of FIGs. 9A-11 with one or more of the aforementioned strain reduction features. The end effector 100' includes a framework 120 having a plurality of spines 124 extending along a longitudinal axis 60. For example, the framework 120 can have, but is not limited to, a similar configuration as that of FIG. 3A. The end effector 100' further includes two flexible circuits 110', 150' that oppose one another relative to the framework 120, similar to the flexible circuits 110, 150 of the previously described examples. The flexible circuit 110' generally includes a substrate layer 110A' formed from a stretchable polymer material (e.g., TPU, as discussed above), electrodes 160 connected to the substrate layer 110', conductive traces 111' (FIG. 9B) connected to the substrate layer 110A' that conduct current to/from the electrodes 160, one or more of the aforementioned strain reduction features (with respect to FIGs. 9A-11), and an insulative material 130 at least partially enveloping the framework 120 and the flexible circuit 110'. As will be appreciated by those skilled in the art, the other flexible circuit 150' can be similarly or identically configured as flexible circuit 110'. In some examples, the flexible circuits 110', 150' and the framework are suspended in the insulative material 130 such that the framework 120 is spaced apart, along vertical axis 62 that is perpendicular to longitudinal axis 60, from the flexible circuits 110', 150'.

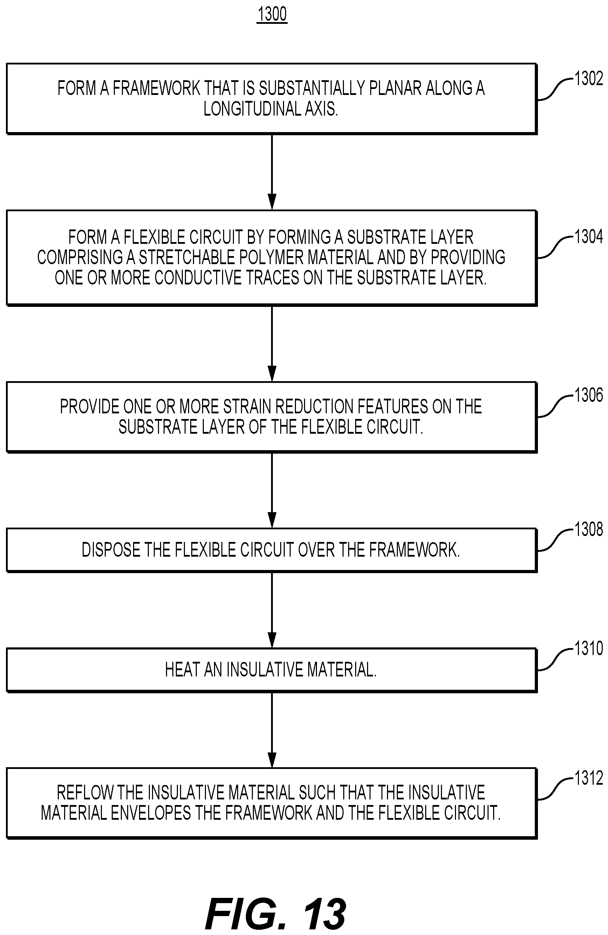

FIG. 13 depicts a flow chart of an exemplary method 1300 of forming an end effector with a flexible circuit includes a stretchable substrate. A framework is formed 1302 that is substantially planar along a longitudinal axis. A flexible circuit is formed 1304 by (i) forming a substrate layer made from a stretchable polymer material (e.g., TPU) and (ii) providing one or more conductive traces (having, e.g., a serpentine shape) on the substrate layer. One or more strain reduction features are provided 1306 on the substrate layer of the flexible circuit. The flexible circuit is disposed 1308 over the framework. An insulative material (e.g., TPU) is heated 1310 and reflowed 1312 such that the insulative material envelopes the framework and the flexible circuit.

In some examples, the forming 1304 of the flexible circuit step can further include providing one or more electrodes on the substrate layer and connecting the one or more conductive traces to the one or more electrodes. In this example, the electrodes can be exposed through the reflowed insulative material by, for example, a laser exposing process.

In some examples, the step of providing one or more conductive traces on the substrate layer can include the following. A conductive material is deposited on the substrate layer. A layer of photoresist is applied to the conductive material. A photomask is aligned over the layer of photoresist, with the photomask having a conductive trace pattern. The substrate layer is exposed to ultraviolet light through the photomask. Portions of the conductive material are then removed to form the one or more conductive traces with the remaining portions not removed.

In some examples, the step of providing one or more conductive traces on the substrate layer can printing conductive ink onto the substrate layer to form the one or more conductive traces.

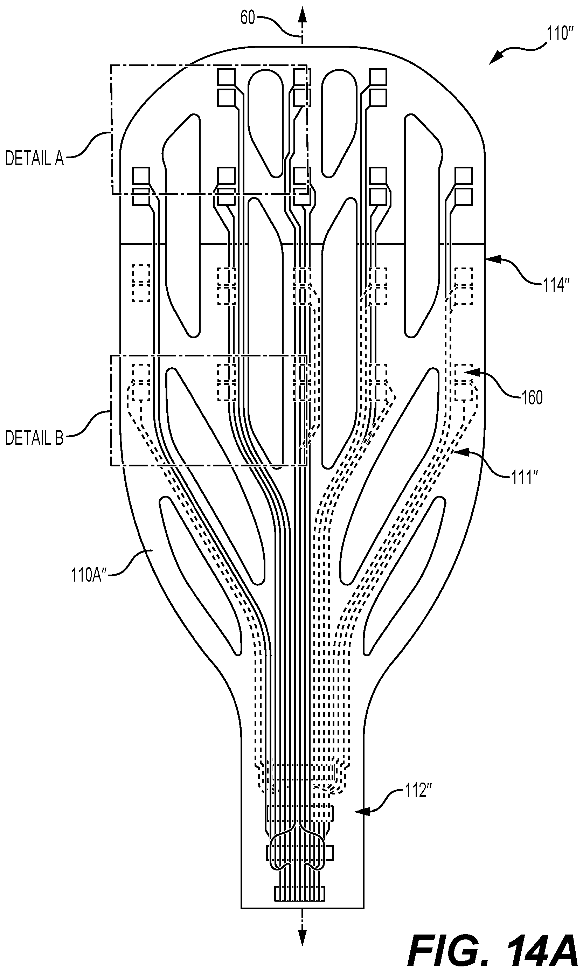

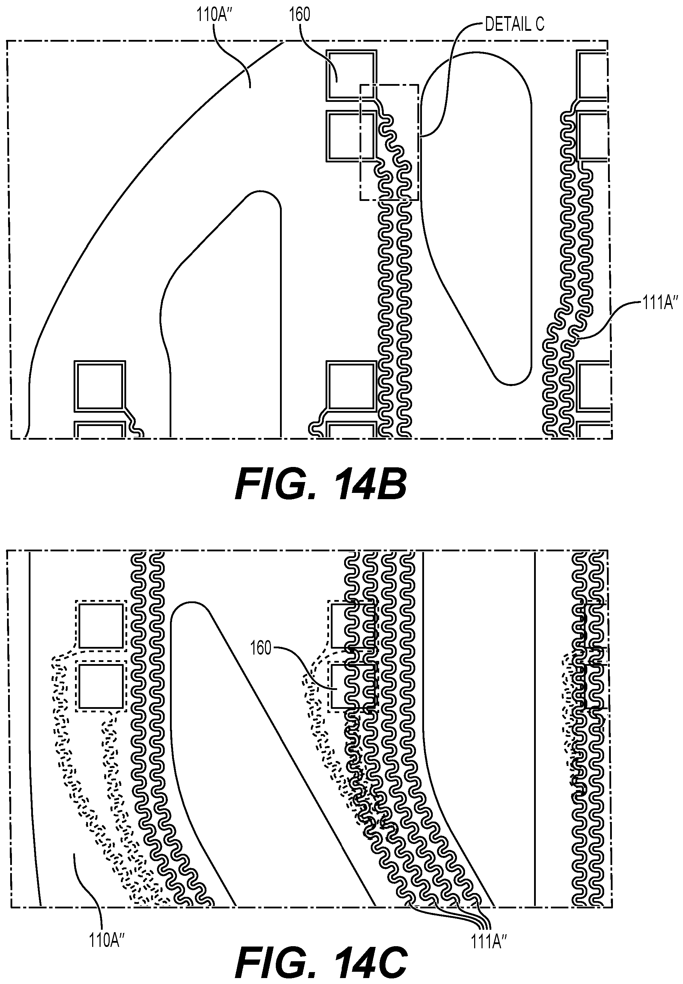

FIGs. 14A-14D depicts various views of another exemplary flexible circuit 110" that includes unnested micro-serpentine traces 111". FIG. 14A is a schematic layout of the entire flexible circuit 110", with FIGs. 14B-14D being respective detail views thereof. With reference to FIG. 14A, the flexible circuit 110", like previous examples, extends along a longitudinal axis 60 and includes at least a substrate layer 110A" and multiple conductive traces 111". The flexible circuit 110" and substrate layer 110A" have a base 112" and multiple of tines 114" extending from the base 112", as in previously described examples. The traces 111" extend from the base 112" and along respective tines 114", with the tines 114" each typically including multiple traces 111" running there along. The substrate layer 110A" can include, but is not limited to, any of the stretchable and/or soft polymers previously described with respect to FIGs. 8A-11. Moreover, any of the aforementioned strain reduction features (FIGs. 9A-11) can be used in the configuration of flexible circuit 110" depicted in FIGs. 14A-14D.It is further noted that the traces 111" shown in phantom in FIGs. 14A and 14C denote traces 111" on a different substrate layer than the traces 111" shown in solid lines.

The conductive traces 111", which conduct current, generally extend along the longitudinal axis 60 and are connected to the substrate layer 110A". As seen particularly in FIGs. 14B-14C, the traces 111" have a serpentine shape and run generally parallel to each other but in a non-overlapping manner (relative to any given cross-sectional cut taken along the longitudinal axis 60). It is noted that this example can incorporate certain features of the previously described examples (e.g., a stretchable substrate layer, strain reduction feature(s), etc.). Each trace 111" is routed and connected to an electrode 160 disposed on the substrate layer 110A".

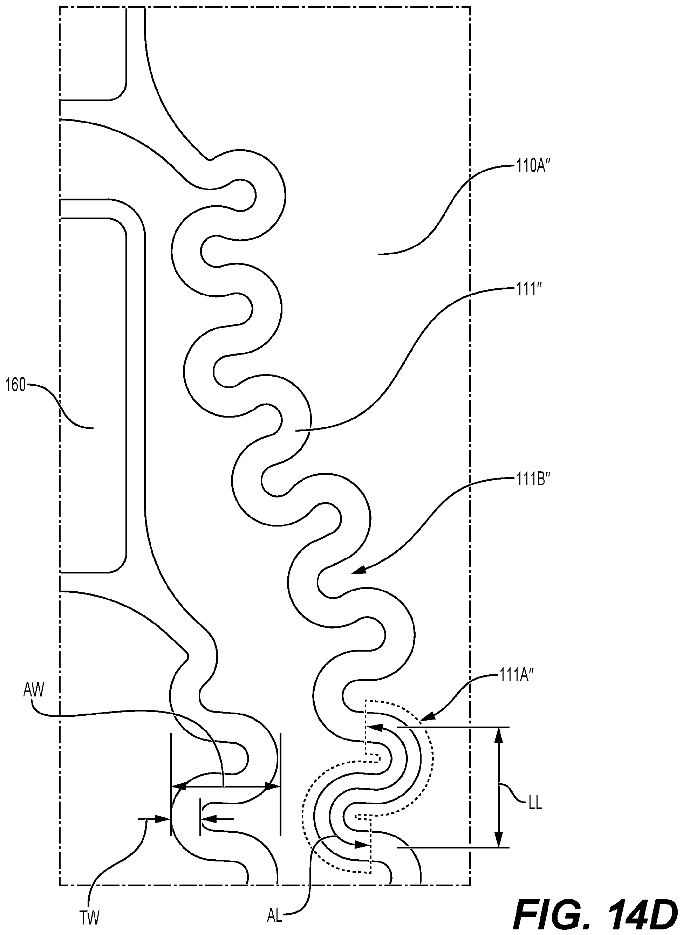

Further to the above, and as seen particularly in FIG. 14D, each conductive trace has an undulating serpentine shape that can be subdivided into multiple arc sections 111A" (denoted by the dashed boundary line for the purposes of illustration) connected to one another along the longitudinal axis 60. Each arc section 111A" has a S-shape or a reversed S-shape such that a pair of voids 111B" are defined. The voids 111B" of each arc section 111A", due to the serpentine shape of the traces 111", have open ends that face in opposite directions.

As shown in FIGs. 14A-14D, Each conductive trace 111" generally extends along the longitudinal axis 60 and has one or more portions/regions that run adjacent to one or more other conductive traces 111" with a non-overlapping/unnested relationship. Put another way, each arc section 111A" of any respective conductive trace 111" is laterally spaced from the voids 111B" of the arc sections 111A" of any adjacent conductive traces 111". Each arc section 111A" is also non-concentric relative to an adjacent arc section 111A" of an adjacent trace 111". With this configuration, the radii of the curves of the arc sections 111A" can be preserved. If they were to be nested (i.e., include adjacent sections that are concentric, or in other words, with the curve of one trace 111" concentric to an adjacent one), the radius of the adjacent trace 111" would necessarily be smaller, significantly increasing the stress and breakability thereof.

Each arc section 111A" has a linear length LL and an arc length AL that is greater than the linear length. As seen in FIG. 14D, the arc length is defined as the path distance from the midpoint (relative to the trace width TW) of the distal end of the arc section 111A" to the midpoint (relative to the trace width TW) of the proximal end of the arc section 111A". In some examples, a ratio of the arc length AL to the linear length LL is approximately two to one. In some examples, each arc length has a value in a range of approximately .26 millimeters to .32 millimeters. In some examples, each linear length LL has a value in a range of approximately .13 millimeters to .17 millimeters. In some examples, each arc section 111A" has an arc section width AW (i.e., a total/overall width of the arc section 111A") with a value in a range of approximately .12 millimeters to .16 millimeters. In some example, each conductive trace 111" has a trace width TW with a value in a range of approximately .03 millimeters to .05 millimeters.

The disclosed technology described herein can be further understood according to the following clauses:
Clause 1. A framework for an end effector of a medical device, the framework comprising: base configured to connect with an elongated shaft of the medical device, the base extending along a longitudinal axis in a distal direction; a first cantilevered spine extending from the base along the longitudinal axis to a terminal portion; a second cantilevered spine extending from the base along the longitudinal axis to a terminal portion; and a first support connecting the terminal portion of the first cantilevered spine with an intermediate portion of the second cantilevered spine.
Clause 2. The framework of clause 1, the terminal portion of the first cantilevered spine comprising a first eyelet.
Clause 3. The framework of clause 2, the first eyelet of the first cantilevered spine comprising an elongated shape.
Clause 4. The framework of any one of clauses 2-3, the first eyelet of the first cantilevered spine comprising a distal end of the first cantilevered spine.
Clause 5. The framework of any one of clauses 1-4, the second cantilevered spine comprising a first eyelet.
Clause 6. The framework of clause 5, the first eyelet of the second cantilevered spine comprising an elongated shape.
Clause 7. The framework of any one of clauses 5-6, the first support connecting the first eyelet of the first cantilevered spine and the first eyelet of the second cantilevered spine.
Clause 8. The framework of clause 5 or clause 7, the second cantilevered spine comprising a second eyelet, the second eyelet comprising a distal end of the second cantilevered spine.
Clause 9. The framework of any one of clauses 1-8, further comprising a third cantilevered spine extending from the base along the longitudinal axis.
Clause 10. The framework of clause 9, further comprising a second support connecting a terminal portion of the second cantilevered spine with an intermediate portion of the third cantilevered spine.
Clause 11. The framework of any one of clauses 9-10, the third cantilevered spine comprising an elongated eyelet, the elongated eyelet overlapping with the first eyelet and the second eyelet of the second cantilevered spine in the distal direction.
Clause 12. The framework of any one of clauses 1-11, further comprising a fourth cantilevered spine extending from the base along the longitudinal axis.
Clause 13. The framework of clause 12, further comprising a third support connecting an intermediate portion of the third cantilevered spine with a terminal portion of the fourth cantilevered spine.
Clause 14. The framework of any one of clauses 1-13, further comprising a fifth cantilevered spine extending from the base along the longitudinal axis.
Clause 15. The framework of clause 14, further comprising a fourth support connecting an intermediate portion of the fourth cantilevered spine with a terminal portion of the fifth cantilevered spine.
Clause 16. The framework of clause 15, the first support and the second support extending substantially parallel to one another, and the third support and the fourth support extending substantially parallel to one another.
Clause 17. The framework of any one of clauses 1-16, the framework being symmetric relative to the longitudinal axis.
Clause 18. The framework of any one of clauses 1-17, the first support comprising a pair of segments that each connect the terminal portion of the first cantilevered spine with the intermediate portion of the second cantilevered spine.
Clause 19. The framework of any one of clauses 1-18, the first support extending (i) at a non-perpendicular angle relative to the longitudinal axis and (ii) in the distal direction from the second cantilevered spine to the first cantilevered spine.
Clause 20. The framework of any one of clauses 1-19, the second cantilevered spine comprising a segment that extends substantially parallel to the first support.
Clause 21. The framework of any one of clauses 1-20, the first support comprising a pair of first supports, each first support comprising a pair of segments.
Clause 22. The framework of any one of clauses 1-21, comprising a material with super-elastic properties.
Clause 23. An end effector for a catheter, the end effector comprising: a framework comprising a base and a plurality of spines extending from the base along a longitudinal axis; and a flexible circuit comprising: a plurality of segments extending along the longitudinal axis, the plurality of segments defining an area not covered by any material; a plurality of electrodes disposed on each of the segments; and an insulative material disposed between the framework and the flexible circuit so that the framework is spaced apart from the flexible circuit.
Clause 24. The end effector of clause 23, further comprising a position sensor comprising a plurality of location sensing loops.
Clause 25. The end effector of clause 24, the plurality of location sensing loops comprising: a pair of side loops disposed generally symmetrical about the longitudinal axis, each side loop extending in a loop from a proximal portion of the insulative material, to a distal portion of the insulative material, and back to the proximal portion of the insulative material along the longitudinal axis.
Clause 26. The end effector of any one of clauses 24-25, the plurality of location sensing loops comprising: a center loop disposed over the longitudinal axis over an area near a distal portion of the insulative material.
Clause 27. The end effector of clause 26, the center loop partially overlapping with each side loop.
Clause 28. The end effector of any one of clauses 24-27, the position sensor comprising: a plurality of segments that comprise a first segment and a second segment; and a plurality of curved connecting portions that comprise a first curved connecting portion having an arc shape with an angle equal to or greater than one-hundred and eighty degrees and connecting the first segment and second segment.
Clause 29. The end effector of any clause 28, the framework comprising a plurality of eyelets, each curved connecting portion being aligned with a respective eyelet along a vertical direction of the end effector.
Clause 30. The end effector of any one of clauses 28-29, the plurality of segments of the position sensor comprising a plurality of linear segments, and the framework comprising a plurality of supports, each support (i) connecting a pair of adjacent spines and (ii) extending substantially parallel to and aligned with, in a vertical direction, and a respective linear segment of the plurality of linear segments.
Clause 31. The end effector of any one of clauses 24-30, a distalmost end of the position sensor being spaced from a distalmost end of the framework by a predetermined distance.
Clause 32. The end effector of any one of clauses 23-31, further comprising: another flexible circuit disposed generally parallel to the framework and separated from the framework by the insulative material, the another flexible circuit including a plurality of segments; and a plurality of electrodes disposed on the plurality of segments of the another flexible circuit.
Clause 33. The end effector of any one of clauses 23-31, the electrodes on each segment of the flexible circuit being aligned with other electrodes on the other segments of the flexible circuit along the longitudinal axis.
Clause 34. The end effector of one of clauses 23-31, the electrodes on each segment of the flexible circuit being alternatingly staggered with other electrodes on the other segments of the flexible circuit along the longitudinal axis.
Clause 35. The end effector of any one of clauses 24-34, further comprising a bridge that (i) forms a distalmost portion of the position sensor and (ii) connects to some of the plurality of curved connecting portions.
Clause 36. An end effector for a catheter, the end effector comprising: a framework comprising a base and a plurality of spines extending from the base along a longitudinal axis; and a position sensor spaced apart from the framework the position sensor comprising a plurality of location sensing loops comprising: a pair of side loops disposed generally symmetrical about the longitudinal axis, each side loop extending in a loop from a proximal portion of the framework, to a distal portion of the framework, and back to the proximal portion of the framework along the longitudinal axis.
Clause 37. The end effector of clause 36, further comprising an insulative material disposed between the framework and the position sensor, and wherein the plurality of location sensing loops further comprising: a center loop disposed over the longitudinal axis over an area near a distal portion of the insulative material.
Clause 38. The end effector of clause 37, the center loop partially overlapping with each side loop.
Clause 39. The end effector of any one of clauses 36-38, the position sensor comprising a plurality of side segments and a center segment, each side loop running along the center segment.
Clause 40. The end effector of any one of clauses 36-39, further comprising : a flexible circuit disposed generally parallel to the framework and separated from the framework by the insulative material, the flexible circuit including a plurality of segments; and a plurality of electrodes disposed on the plurality of segments of the flexible circuit.
Clause 41. The end effector of clause 40, the electrodes on each segment of the flexible circuit being aligned with other electrodes on the other segments of the flexible circuit along the longitudinal axis.
Clause 42. The end effector of clause 40, the electrodes on each segment of the flexible circuit being alternatingly staggered with other electrodes on the other segments of the flexible circuit along the longitudinal axis.
Clause 43. A flexible circuit comprising: a substrate layer comprising a stretchable polymer material; one or more conductive traces connected to the substrate layer and configured to conduct current; and one or more strain reduction features connected to the substrate layer, each strain reduction feature comprising one of: a sacrificial trace connected to the substrate layer; a heat sink connected to a base of the substrate layer; or a localized stiffener connected to the substrate layer in a region in which a portion of the one or more conductive traces extend.
Clause 44. The flexible circuit of clause 43, the one or more strain reduction features comprising the sacrificial trace, the sacrificial trace extending in one of an undulating pattern or a substantially linearly direction.
Clause 45. The flexible circuit of clause 44, the sacrificial trace extending in the undulating pattern, the undulating pattern comprising a same undulating pattern as the one or more conductive traces.
Clause 46. The flexible circuit of clause 44, the sacrificial trace extending in the undulating pattern, the undulating pattern comprising a lower frequency of oscillations than an undulating pattern of the one or more traces.
Clause 47. The flexible circuit of any one of clauses 44-46, the one or more conductive traces being connected to a first surface of the substrate layer, and the sacrificial trace being connected to an opposing second surface of the substrate layer.
Clause 48. The flexible circuit of any one of clauses 43-47, the substrate layer further comprising the base, the base comprising a soldering pad region, and the one or more strain reduction features comprising the heat sink, the heat sink being configured to dissipate heat from the soldering pad region.
Clause 49. The flexible circuit of clause 48, the soldering pad region being connected to a first surface of the substrate layer, and the heat sink being connected to an opposing second surface of the substrate layer.
Clause 50. The flexible circuit of any one of clauses 43-49, the one or more strain reduction features comprising the localized stiffener, the localized stiffener comprising one of a metal plate or a polyimide pad.
Clause 51. The flexible circuit of any one of clauses 43-50, the one or more conductive traces comprising a serpentine shape.
Clause 52. The flexible circuit of any one of clauses 43-51, the stretchable polymer material comprising flexibility in three dimensions.
Clause 53. The flexible circuit of any one of clauses 43-52, the stretchable polymer material comprising thermoplastic polyurethane.
Clause 54. The flexible circuit of any one of clauses 43-53, further comprising a plurality of electrodes connected to the substrate layer, each conductive trace of the one or more conductive traces being connected to a respective electrode of the plurality of electrodes.
Clause 55. An end effector for a catheter, the end effector comprising: a framework comprising a plurality of spines extending along a longitudinal axis; a flexible circuit comprising: a substrate layer comprising a stretchable polymer material; a plurality of conductive traces connected to the substrate layer and configured to conduct current; a plurality of electrodes connected to the substrate layer, a respective conductive trace of the plurality of conductive traces being connected to a respective electrode of the plurality of electrodes; one or more strain reduction features connected to the substrate layer, each strain reduction feature comprising one of: a sacrificial trace connected to the substrate layer; a heat sink connected to a base of the substrate layer; or a localized stiffener connected to the substrate layer in a region in which a portion of one or more of the plurality of conductive traces extend; and an insulative material at least partially enveloping the framework and the flexible circuit.
Clause 56. The end effector of clause 55, the flexible circuit and the framework being suspended in the insulative material such that the framework is spaced apart from the flexible circuit.
Clause 57. The end effector of any one of clauses 54-56, the one or more strain reduction features comprising the sacrificial trace, the sacrificial trace extending in one of an undulating pattern or a substantially linearly direction.
Clause 58. The end effector of clause 57, the sacrificial trace extending in the undulating pattern, the undulating pattern comprising a same undulating pattern as the one or more conductive traces.
Clause 59. The end effector of clause 57, the sacrificial trace extending in the undulating pattern, the undulating pattern comprising a lower frequency of oscillations than an undulating pattern of the one or more traces.
Clause 60. The end effector of any one of clauses 57-59, the one or more conductive traces being connected to a first surface of the substrate layer, the sacrificial trace, and the sacrificial trace being connected to an opposing second surface of the substrate layer.
Clause 61. The end effector of any one of clauses 55-60, the substrate layer further comprising the base, the base comprising a soldering pad region, and the one or more strain reduction features comprising the heat sink, the heat sink being configured to dissipate heat from the soldering pad region.
Clause 62. The end effector of clause 61, the soldering pad region being connected to a first surface of the substrate layer, and the heat sink being connected to an opposing second surface of the substrate layer.
Clause 63. The end effector of any one of clauses 55-62, the one or more strain reduction features comprising the localized stiffener, the localized stiffener comprising one of a metal plate or a polyimide pad.
Clause 64. The end effector of any one of clauses 55-63, the one or more conductive traces comprising a serpentine shape.
Clause 65. The end effector of any one of clauses 55-64, the stretchable polymer material comprising flexibility in three dimensions.
Clause 66. The end effector of any one of clauses 55-65, the stretchable polymer material comprising thermoplastic polyurethane.
Clause 67. A method of manufacturing an end effector for a medical device, the method comprising: forming a framework that is substantially planar along a longitudinal axis; forming a flexible circuit comprising: forming a substrate layer comprising a stretchable polymer material; and providing one or more conductive traces on the substrate layer; providing one or more strain reduction features on the substrate layer of the flexible circuit; disposing the flexible circuit over the framework; heating an insulative material; and reflowing the insulative material such that the insulative material envelopes the framework and the flexible circuit.
Clause 68. The method of clause 67, the forming a flexible circuit further comprising: providing one or more electrodes on the substrate layer; and connecting the one or more conductive traces to the one or more electrodes.
Clause 69. The method of clause 68, further comprising: laser exposing the one or more electrodes through the reflowed insulative material.
Clause 70. The method of any one of clauses 67-69, the stretchable polymer material comprising thermoplastic polyurethane.
Clause 71. The method of any one of clauses 67-70, the insulative material comprising thermoplastic polyurethane.
Clause 72. The method of any one of clauses 67-71, the providing one or more conductive traces on the substrate layer comprising: depositing a conductive material on the substrate layer; applying a layer of photoresist to the conductive material; aligning a photomask over the layer of photoresist, the photomask comprising a conductive trace pattern; exposing the substrate layer to ultraviolet light through the photomask; and removing portions of the conductive material to form the one or more conductive traces.
Clause 73. The method of any one of clauses 67-71, the providing one or more conductive traces on the substrate layer comprising: printing conductive ink onto the substrate layer to form the one or more conductive traces.
Clause 75. The method of any one of clauses 67-73, the one or more conductive traces comprising a serpentine shape.
Clause 76. A flexible circuit extending along a longitudinal axis and comprising: a substrate layer; and a plurality of conductive traces connected to the substrate layer and configured to conduct current, each conductive trace comprising a serpentine shape comprising a plurality of arc sections, each arc section defining a pair of voids with oppositely facing open ends, and each conductive trace generally extending along the longitudinal axis adjacent to one or more conductive traces of the plurality of conductive traces such that each arc section of any respective conductive trace of the plurality of traces is laterally spaced from the voids of the arc sections of the adjacent one or more conductive traces.
Clause 77. The flexible circuit of clause 76, further comprising a plurality of electrodes connected to the substrate layer, each conductive trace being connected to a respective electrode of the plurality of electrodes.
Clause 78. The flexible circuit of any one of clauses 76-77, the substrate layer comprising a base and a plurality of tines extending from the base, the plurality of conductive traces extending from the base and along the plurality of tines.
Clause 79. The flexible circuit of any one of clauses 76-78, each arc section comprising a linear length and an arc length that is greater than the linear length.
Clause 80. The flexible circuit of clause 79, a ratio of the arc length to the linear length being approximately two to one.
Clause 81. The flexible circuit of any one of clauses 79-80, each arc length being in a range of approximately .26 millimeters to .32 millimeters.
Clause 82. The flexible circuit of any one of clauses 79-81, each linear length being in a range of approximately .13 millimeters to .17 millimeters.
Clause 83. The flexible circuit of any one of clauses 76-82, each arc section comprising a linear width in a range of approximately .12 millimeters to .16 millimeters.
Clause 84. The flexible circuit of any one of clauses 76-83, each conductive trace comprising a trace width in a range of approximately .03 millimeters to .05 millimeters.
The examples described above are cited by way of example, and the disclosed technology is not limited by what has been particularly shown and described hereinabove. Rather, the scope of the disclosed technology includes both combinations and sub combinations of the various features described hereinabove, as well as variations and modifications thereof which would occur to persons skilled in the art upon reading the foregoing description and which are not disclosed in the prior art.

## Claims

1. A framework for an end effector of a medical device, the framework comprising:
a base configured to connect with an elongated shaft of the medical device, the base extending along a longitudinal axis in a distal direction;
a first cantilevered spine extending from the base along the longitudinal axis to a terminal portion;
a second cantilevered spine extending from the base along the longitudinal axis to a terminal portion; and
a first support connecting the terminal portion of the first cantilevered spine with an intermediate portion of the second cantilevered spine.

2. The framework of claim 1, the terminal portion of the first cantilevered spine comprising a first eyelet.

3. The framework of claim 2, the first eyelet of the first cantilevered spine comprising an elongated shape, or comprising a distal end of the first cantilevered spine.

4. The framework of claim 1, the second cantilevered spine comprising a first eyelet.

5. The framework of claim 1, further comprising a third cantilevered spine extending from the base along the longitudinal axis, optionally further comprising a fourth cantilevered spine extending from the base along the longitudinal axis, further optionally further comprising a fifth cantilevered spine extending from the base along the longitudinal axis.

6. The framework of claim 1, the first support comprising a pair of segments that each connect the terminal portion of the first cantilevered spine with the intermediate portion of the second cantilevered spine.

7. The framework of claim 1, the first support extending (i) at a non-perpendicular angle relative to the longitudinal axis and (ii) in the distal direction from the second cantilevered spine to the first cantilevered spine.

8. An end effector for a catheter, the end effector comprising:
a framework comprising a base and a plurality of spines extending from the base along a longitudinal axis;
a flexible circuit comprising:
a plurality of segments extending along the longitudinal axis, the plurality of segments defining an area not covered by any material; and
a plurality of electrodes disposed on each of the segments; and
an insulative material disposed between the framework and the flexible circuit so that the framework is spaced apart from the flexible circuit.

9. The end effector of claim 8, further comprising a position sensor comprising a plurality of location sensing loops.

10. The end effector of claim 9, the plurality of location sensing loops comprising:
i) a pair of side loops disposed generally symmetrical about the longitudinal axis, each side loop extending in a loop from a proximal portion of the insulative material, to a distal portion of the insulative material, and back to the proximal portion of the insulative material along the longitudinal axis, and/or
ii) a center loop disposed over the longitudinal axis over an area near a distal portion of the insulative material, optionally the center loop partially overlapping with each side loop.

11. An end effector for a catheter, the end effector comprising:
a framework comprising a base and a plurality of spines extending from the base along a longitudinal axis; and
a position sensor spaced apart from the framework the position sensor comprising a plurality of location sensing loops comprising:
a pair of side loops disposed generally symmetrical about the longitudinal axis, each side loop extending in a loop from a proximal portion of the framework, to a distal portion of the framework, and back to the proximal portion of the framework along the longitudinal axis.

12. The end effector of claim 11, further comprising an insulative material disposed between the framework and the position sensor, and wherein the plurality of location sensing loops further comprising:
a center loop disposed over the longitudinal axis over an area near a distal portion of the insulative material.

13. The end effector of claim 12, the center loop partially overlapping with each side loop.

14. The end effector of claim 11, the position sensor comprising a plurality of side segments and a center segment, each side loop running along the center segment.

15. The end effector of claim 12, further comprising:
a flexible circuit disposed generally parallel to the framework and separated from the framework by the insulative material, the flexible circuit including a plurality of segments; and
a plurality of electrodes disposed on the plurality of segments of the flexible circuit.
